# EUROPÄISCHE PATENTANMELDUNG

(11) **EP 4 749 662 A1**
(43) Veröffentlichungstag der Anmeldung: **27.05.2026**
(21) Anmeldenummer: 25166000.7
(22) Anmeldetag: 25.03.2025
(51) Int. Cl.: G21K 1/02, A61N 5/10

(54) **SCHICHTFÖRMIGE ABSORBERMASKE FÜR DIE STRAHLENTHERAPIE**

(30) Priorität: 22.11.2024 EP 24214811; 23.12.2024 EP 24222839
(71) Anmelder: Siemens Healthineers AG, 91301 Forchheim (DE)
(72) Erfinder: Bräuer, Martin, 90427 Nürnberg (DE)
(74) Vertreter: Siemens Healthineers Patent Attorneys

(57) **Zusammenfassung**

Die erfindungsgemäße Absorbermaske zum Formen einer Vielzahl von therapeutischen Röntgenstrahlen, insbesondere für eine Strahlentherapie eines Patienten, weist
- einen Schichtstapel auf,
- wobei der Schichtstapel mehrere Röntgenstrahlen-absorbierende Schichten aufweist,
- wobei die mehreren Röntgenstrahlen-absorbierenden Schichten jeweils mindestens eine flächige Lage aufweisen,
- wobei benachbarte Lagen des Schichtstapels mittels eines additiven Herstellungsverfahrens in Stapelrichtung flächig aufeinander aufgetragen sind,
- wobei zwischen den mehreren Röntgenstrahlen-absorbierenden Schichten mindestens eine Röntgenstrahlen-transparente Schicht und/oder mindestens ein sich in Breitenrichtung erstreckender Röntgenstrahlen-absorbierender Streifen angeordnet ist.

## Beschreibung

Die Erfindung betrifft eine Absorbermaske, eine Anordnung für eine Strahlentherapie eines Patienten, ein Verfahren zur Herstellung einer Absorbermaske zum Formen einer Vielzahl von therapeutischen Röntgenstrahlen und eine Anlage zur additiven Herstellung einer Absorbermaske zum Formen einer Vielzahl von therapeutischen Röntgenstrahlen.

Die vorliegende Erfindung bezieht sich auf das Gebiet der Strahlentherapie. Strahlentherapie ist eine weit verbreitete Methode zur therapeutischen Behandlung von beispielsweise Krebs und anderen Krankheiten, bei der therapeutische Strahlung, insbesondere therapeutische Röntgenstrahlung, als ionisierende Strahlung verwendet wird, um bösartige Zellen an der Teilung zu hindern

Nach dem aktuellen Stand der Technik werden Absorbermasken zum Formen einer Vielzahl von therapeutischen Röntgenstrahlen mechanisch aus Metallblöcken oder Blechen hergestellt. Wolfram ist aufgrund seiner extrem hohen Dichte und hoher Abschirmwirkung bei gegebener Dichte ein bevorzugtes Material für diese Absorbermasken. Allerdings wird Wolfram selten verwendet, da die mechanische Bearbeitung äußerst schwierig und teuer ist. Stattdessen werden im Stand der Technik häufig andere Metalle wie Messing oder Eisen verwendet, die sich mechanisch viel besser bearbeiten lassen, obwohl sie eine deutlich geringere Abschirmwirkung haben. In einigen Fällen werden die Masken auch additiv gefertigt, beispielsweise durch 3D-Druckverfahren. Hierbei kommen Verfahren zum Einsatz, bei denen Metallpulver verfestigt wird, wie zum Beispiel durch das selektive Laserschmelzen (SLM).

Die Herstellung von Absorbermasken mit sehr feinen Strukturen ist mit konventionellen Methoden schwierig und kostspielig. Zudem sind die verwendeten Materialien oft nicht optimal auf die spezifischen Anforderungen verschiedener Anwendungen der Strahlentherapie abgestimmt. Die mangelnde Flexibilität in der Gestaltung der Absorbermasken führt zu Kompromissen zwischen Dosismaximierung im Zielbereich und Schonung des umliegenden Gewebes.

Der Erfindung liegt die Aufgabe zu Grunde, eine Absorbermaske, eine Anordnung für eine Strahlentherapie eines Patienten, ein Verfahren zur Herstellung einer Absorbermaske zum Formen einer Vielzahl von therapeutischen Röntgenstrahlen und eine Anlage zur additiven Herstellung einer Absorbermaske zum Formen einer Vielzahl von therapeutischen Röntgenstrahlen anzugeben, welche verbessert, insbesondere flexibler, vorzugsweise patientenindividuell herstellbar, sind.

Die Aufgabe wird durch die Merkmale der unabhängigen Ansprüche gelöst. Vorteilhafte Ausgestaltungen sind in den Unteransprüchen beschrieben.

Die erfindungsgemäße Absorbermaske zum Formen einer Vielzahl von therapeutischen Röntgenstrahlen, insbesondere für eine Strahlentherapie eines Patienten, weist
- einen Schichtstapel auf,
- wobei der Schichtstapel mehrere Röntgenstrahlen-absorbierende Schichten aufweist,
- wobei die mehreren Röntgenstrahlen-absorbierenden Schichten jeweils mindestens eine flächige Lage aufweisen,
- wobei benachbarte Lagen des Schichtstapels mittels eines additiven Herstellungsverfahrens in Stapelrichtung flächig aufeinander aufgetragen sind,
- wobei zwischen den mehreren Röntgenstrahlen-absorbierenden Schichten mindestens eine Röntgenstrahlen-transparente Schicht und/oder mindestens ein sich in Breitenrichtung erstreckender Röntgenstrahlen-absorbierender Streifen angeordnet ist.

Ein erfindungsgemäßes Verfahren zur Herstellung einer Absorbermaske zum Formen einer Vielzahl von therapeutischen Röntgenstrahlen, insbesondere für eine Strahlentherapie eines Patienten, umfasst die Schritte:
- Auftragen mittels eines additiven Herstellungsverfahrens von Lagen flächig aufeinander in Stapelrichtung zu einem Schichtstapel derart,
- dass der Schichtstapel mehrere Röntgenstrahlen-absorbierende Schichten aufweist und
- dass der Schichtstapel zwischen den mehreren Röntgenstrahlen-absorbierenden Schichten mindestens eine Röntgenstrahlen-transparente Schicht und/oder mindestens einen sich in Breitenrichtung erstreckenden Röntgenstrahlen-absorbierenden Streifen aufweist.

Der erfindungsgemäße Schritt der vorliegenden Erfindung besteht in einer innovativen Betrachtung der Richtung der additiven Fertigung. Denn die Lagen der (optionalen) Röntgenstrahlen-transparenten und Röntgenstrahlen-absorbierenden Schichten werden in Stapelrichtung flächig aufeinander aufgetragen. Die Absorbermaske wird also gefertigt, indem viele Lagen übereinander zu einem Schichtstapel aufgetragen werden. Vorteilhafterweise werden die Absorbermasken demnach gefertigt, indem eine erfindungsgemäße Anlage zur additiven Herstellung einer Absorbermaske so ertüchtigt und eingesetzt wird, dass dünne Lagen von minimaler Dicke aufgetragen werden. Dazu wird die entstehende Absorbermaske beispielsweise auf einer vertikal beweglichen Platte, die im Verlauf des Fertigungsprozesses abgesenkt und/oder horizontal bewegt wird, aufgebaut. Eine solche Anordnung ist aus dem Bereich der additiven Herstellungsverfahren, z.B. aus dem 3D-Druck, insbesondere als "core xy"-Anordnung bekannt. Grundsätzlich ist es denkbar, aber nicht Teil der Erfindung, die Fertigungsrichtung senkrecht zur Oberseite einzustellen.

Eine Absorbermaske, regelmäßig auch als Absorberraster oder Absorbergitter bezeichnet, dient dazu, die therapeutische Röntgenstrahlung auf das zu therapierende Gewebe des Patienten auszurichten. Dafür formt die Absorbermaske eine Vielzahl an therapeutischen Röntgenstrahlen, indem Röntgenstrahlen-absorbierendes Material zu schonendes Gewebe abschattet. Öffnungen zwischen dem Röntgenstrahlen-absorbierenden Material, welche mit Röntgenstrahlen-transparentem Material gefüllt oder materialfrei sein können, formen dahingegen ausgehend von der therapeutischen Röntgenstrahlenquelle die Vielzahl an therapeutischen Röntgenstrahlen, welche das Gewebe im Zielbereich mit einer entsprechend hohen Röntgendosis therapieren. Diese therapeutischen Röntgenstrahlen sind die sogenannten "Minibeams" oder "Microbeams" oder auch primären Röntgenstrahlen. Ob ein Röntgenphoton mittels des Röntgenstrahlen-absorbierenden Materials absorbiert wird oder als therapeutischer Röntgenstrahl durch die Absorbermaske transmittiert, hängt insbesondere von dem Einfallwinkel der Trajektorie, auf welcher das Röntgenphoton ausgehend von der therapeutischen Röntgenstrahlenquelle sich befindet, in Bezug zur Oberfläche der Absorbermaske ab. Die Oberfläche der Absorbermaske ist insbesondere auf der Oberseite der Absorbermaske.

Die Absorbermaske ist insbesondere eine passive Vorrichtung, welche nur dann therapeutische Röntgenstrahlen formen kann, wenn sie entsprechend von einer therapeutischen Röntgenstrahlenquelle bestrahlt wird. Die Absorbermaske selbst erzeugt keine therapeutischen Röntgenstrahlen. Das Formen der Vielzahl an therapeutischen Röntgenstrahlen umfasst insbesondere ein Absorbieren solcher Röntgenstrahlen, welche nicht auf den Patienten auftreffen sollen, und ein Transmittieren lassen derjenigen Röntgenstrahlen, welche nach dem Passieren der Absorbermaske als therapeutische Röntgenstrahlen im Zielbereich im Gewebe des Patienten therapierend wirken sollen.

Therapeutische Röntgenstrahlung ist insbesondere dadurch gekennzeichnet, dass die Röntgenphotonen eine Energie von typischerweise bis zu 80 keV, beispielsweise bis zu 140 keV, je nach Anwendungsfall der Absorbermaske, aufweisen. Die Röntgenphotonen weisen typischerweise mindestens 40 keV, vorzugsweise mindestens 80 keV auf. Im Folgenden werden die Begriffe der therapeutischen Röntgenstrahlung und der Röntgenstrahlung als Synonyme verwendet.

In der vorliegenden Anmeldung werden die Ausdehnungen einer Schicht bzw. einer Lage wie folgt bezeichnet: In Hinblick auf das additive Fertigungsverfahren, bei welchem typischerweise Lage für Lage Material in eine (Haupt-)Auftragungsrichtung aufgetragen wird, bezieht sich die Länge der Lage bzw. der Schicht auf die Ausdehnung in Auftragungsrichtung. Senkrecht dazu ist die Breite der Lage bzw. der Schicht. Das additive Fertigungsverfahren kann dazu ausgebildet sein, senkrecht zur Auftragungsrichtung Material aufzutragen, um die Breite der Lage bzw. der Schicht zu vergrößern. Die Länge und die Breite liegen insbesondere in der Ebene, in welcher die Auftragung erfolgt. Die Dicke, auch als Stärke bezeichnet, der Lage bzw. der Schicht bezeichnet dann die Ausdehnung senkrecht zu dieser Ebene.

Ferner werden die Ausdehnungen der Absorbermaske bzw. des Schichtstapels wie folgt bezeichnet: die Länge der Absorbermaske bzw. des Schichtstapels entspricht dabei typischerweise der Länge der Lage bzw. der Schicht. Die Breite der Absorbermaske bzw. des Schichtstapels ergibt sich näherungsweise aus der Summe der Dicke aller Lagen bzw. der Schicht. Die Breite der Absorbermaske bzw. des Schichtstapels entspricht also im Wesentlichen der Ausdehnung in Stapelrichtung. Die Dicke der Absorbermaske bzw. des Schichtstapels ergibt sich im Wesentlichen aus der Breite der Lage bzw. der Schicht.

Der Schichtstapel bildet die Grundstruktur der Absorbermaske. Ein Schichtstapel bezeichnet eine Anordnung mehrerer übereinander liegender Lagen mit unterschiedlichen Eigenschaften bezüglich der Röntgenstrahlung, insbesondere der Röntgenstrahlungsschwächungseigenschaft. Die Röntgenstrahlungsschwächungseigenschaft definiert die Absorptionsrate von Röntgenstrahlung. Der Schichtstapel ist insbesondere ein Lagenstapel mit mehreren Lagen unterschiedlicher Eigenschaften. Eine Schicht weist insbesondere benachbarte Lagen mit den gleichen Röntgenstrahlungsschwächungseigenschaften auf. Benachbarten Lagen sind insbesondere solche Lagen, welche unmittelbar aufeinander aufgetragen sind. Benachbarte Lagen mit den gleichen Eigenschaften umgeben insbesondere nicht eine oder mehrere Lagen mit einer unterschiedlichen Eigenschaft. Die mindestens eine Lage einer optionalen Röntgenstrahlen-transparenten Schicht und die mindestens eine Lage der mehreren Röntgenstrahlen-absorbierenden Schichten unterscheiden sich insbesondere in ihrer Röntgenstrahlungsschwächungseigenschaft. Die mindestens eine Lage der Röntgenstrahlen-transparenten Schicht weisen insbesondere eine vergleichsweise niedrige Röntgenstrahlungsschwächungseigenschaft und somit eine hohe Röntgenstrahlungstransmissionseigenschaft auf. Die mindestens eine Lage der mehreren Röntgenstrahlen-absorbierenden Schichten weist insbesondere eine vergleichsweise hohe Röntgenstrahlungsschwächungseigenschaft und somit eine niedrige Röntgenstrahlungstransmissionseigenschaft auf.

Die Röntgenstrahlen-transparente Schicht umfasst typischerweise jeweils eine oder mehrere benachbarte Röntgenstrahlen-transparente Lagen und ermöglicht eine hohe Transmission der primären Röntgenstrahlung. Die Röntgenstrahlen-absorbierenden Schichten umfassen mindestens eine oder mehrere benachbarte Röntgenstrahlen-absorbierende Lagen und absorbieren die Röntgenstrahlung möglichst effektiv.

Eine Röntgenstrahlen-transparente Lage kann aus Materialien mit niedriger Ordnungszahl bestehen, beispielsweise aus Kunststoffen wie Polyethylen, Epoxidharz oder Polypropylen. Diese Materialien weisen eine geringe Röntgenstrahlungsschwächungseigenschaft auf und lassen somit einen Großteil der primären Röntgenstrahlung passieren. Alternativ oder zusätzlich können auch Materialien mit geringer Dichte wie Schäume und/oder Aerogele verwendet werden. Eine gute Möglichkeit stellt insbesondere die Verwendung einer Träger-Matrix aus einem Kunststoff, vorzugsweise Polyethylen, dar, der auch häufig in der additiven Fertigung verwendet wird. Dieser Werkstoff Polyethylen eignet sich zudem ohne Anteil eines Röntgenstrahlen-absorbierenden Materials als Röntgenstrahlen-transparentes Material.

Eine Röntgenstrahlen-absorbierende Lage besteht typischerweise aus Materialien mit hoher Ordnungszahl und hoher Dichte. Beispiele hierfür sind Metalle wie insbesondere Blei oder wie vorzugsweise Wolfram, Tantal, Rhenium, Osmium, Iridium, Bismut, Platin, Thallium, Quecksilber oder Gold. Diese Materialien absorbieren Röntgenstrahlung effektiv und können somit die therapeutische Röntgenstrahlungsformung verbessern. Eine Röntgenstrahlen-absorbierende Lage aus Wolfram ist besonders bevorzugt.

Eine flächige Lage bezeichnet eine dünne, ausgedehnte Fläche eines Materials. Die Dicke einer solchen Lage kann im Bereich von wenigen Mikrometern bis zu einigen hundert Mikrometern liegen, während die laterale Ausdehnung deutlich größer ist und typischerweise im Bereich von Zentimetern oder mehr liegt. Die Dicke der Lage beschreibt also eine Stärke der Lage. Eine Lage kann in diesem Zusammenhang als eine einzelne, zusammenhängende Fläche des jeweiligen Materials verstanden werden, die in einem einzigen Fertigungsschritt aufgebracht wird. Die Dicke einer solchen Lage kann je nach verwendetem Material und gewünschten Eigenschaften variieren, zwischen den Lagen und/oder innerhalb der Lage. Beispielsweise kann die Dicke der Röntgenstrahlen-transparenten Lage im Bereich von 20 bis 2000 µm liegen, während die Dicke der Röntgenstrahlen-absorbierenden Lagen im Bereich von 20 bis 1500 µm, insbesondere 20 bis 500 µm, liegen können. Die Dicke einer Lage kann je nach verwendetem Material und/oder gewünschten Eigenschaften variieren. Die Breite der Lage kann insbesondere zwischen 1 mm und 10 cm liegen, vorteilhafterweise zwischen 2 mm und 3 cm. Die Länge der Lage kann insbesondere zwischen 1 cm und 100 cm liegen, beispielsweise zwischen 5 cm und 50 cm.

Das additive Herstellungsverfahren ermöglicht die präzise Fertigung komplexer Strukturen durch schichtweises Auftragen von Material, sprich Lage für Lage. Das additive Herstellungsverfahren erfolgt insbesondere mittels der sogenannten "core xy"-Anordnung als Teil der erfindungsgemäßen Anlage. Bei der Herstellung der Absorbermaske werden die einzelnen Lagen nacheinander aufgebaut, wobei die Lagen für die Röntgenstrahlen-absorbierenden Schichten nacheinander in Stapelrichtung aufgetragen werden. Insbesondere wird die mindestens eine Lage einer der mehreren Röntgenstrahlen-absorbierenden Schichten auf die oberste bzw. letzte der bereits gefertigten Lagen einer der mehreren Röntgenstrahlen-absorbierenden Schichten aufgetragen. Wenn zwischen den mehreren Röntgenstrahlen-absorbierenden Schichten mindestens eine Röntgenstrahlen-transparente Schicht angeordnet ist, wird bei der Auftragung zwischen Röntgenstrahlen-transparentem Material und Röntgenstrahlen-absorbierendem Material abgewechselt. Die Reihenfolge der Fertigung kann auch umgekehrt sein.

Das additive Herstellungsverfahren kann ein schichtweises Aufbauverfahren sein, bei dem die einzelnen Lagen nacheinander aufgetragen werden. Dieses Verfahren kann eine präzise Kontrolle über die Lagendicken und -geometrien ermöglichen. Das Auftragen der Lagen kann durch verschiedene Techniken erfolgen. Eine Möglichkeit kann das selektive Auftragen von Material durch eine Auftragungseinheit, beispielsweise mit einer Auftragungsdüse, sein. Hierbei kann das Material in flüssiger oder pastöser oder Pulverform, insbesondere fest, aufgebracht sein oder anschließend ausgehärtet werden. Eine andere Möglichkeit kann das selektive Verfestigen eines Pulverbetts durch Energieeintrag, beispielsweise durch einen Laser, z.B. durch "selective laser sintering", oder eine andere Energiequelle, sein. Als weitere Methode kann das aufgebrachte Pulver auch durch einen chemischen Prozess, bei dem zusätzliches Material aufgebracht wird, gehärtet werden, beispielsweise durch Auftragen, insbesondere Aufsprühen, einer Flüssigkeit, auch "binder jetting" genannt. Für das Auftragen einer Lage ist es denkbar, dass eine oder beide der Materialzusammensetzungen als Flüssigkeit aufgetragen werden. Insbesondere Röntgenstrahlen-transparente Kunststoffe, wie z.B. Polyethylen, haben typischerweise einen relativ niedrigen Schmelzpunkt von kleiner 300°C, während die Röntgenstrahlen-absorbierenden Materialien, wie beispielsweise Wolfram, vergleichsweise hohe Schmelztemperaturen im Bereich von deutlich oberhalb von 1000°C aufweisen können, z.B. Wolfram 3422°C.

Eine Röntgenstrahlen-transparente Schicht kann eine oder mehrere Röntgenstrahlen-transparente Lagen aufweisen. Die mehreren Röntgenstrahlen-absorbierenden Schichten können jeweils eine oder mehrere Röntgenstrahlen-absorbierende Lagen aufweisen. Es ist denkbar, dass die Anzahl an Lagen je Schicht zwischen den Schichten variiert. Die Anzahl an Lagen je Schicht kann insbesondere 1, 2 oder M > 2 betragen. Vorzugsweise sind die benachbarten Lagen des Schichtstapels, also die jeweiligen aneinander angrenzenden Lagen verschiedener Schichten und die jeweiligen aneinander angrenzenden Lagen innerhalb einer Schicht, mittels des additiven Herstellungsverfahrens in Stapelrichtung flächig aufeinander aufgetragen. Die Dicke einer Schicht kann die Dicke der mindestens einen Lage derjenigen Schicht nicht unterschreiten. Die Dicke einer Schicht beträgt typischerweise mindestens die Summe der Dicken aller Lagen der jeweiligen Schicht.

Es ist bevorzugt, wenn eine Röntgenstrahlen-absorbierende Schicht ausschließlich Röntgenstrahlen-absorbierende Lagen umfasst und wenn eine Röntgenstrahlen-transparente Schicht ausschließlich Röntgenstrahlen-transparente Lagen umfasst. Es ist denkbar, insbesondere in Hinblick auf die Auswahl des Fertigungsverfahrens, dass eine oder mehrere Lagen zwischen einer Röntgenstrahlen-absorbierenden Schicht und einer Röntgenstrahlen-transparenten Schicht eine mittlere Röntgenstrahlungsschwächungseigenschaft aufweisen, beispielsweise auf Grund einer ungewollten Verunreinigung und/oder zur graduellen Einstellung der Röntgenstrahlungsschwächungseigenschaft. Der Betrag der mittleren Röntgenstrahlungsschwächungseigenschaft liegt insbesondere zwischen der niedrigen Röntgenstrahlungsschwächungseigenschaft der Röntgenstrahlen-transparenten Schicht und der hohen Röntgenstrahlungsschwächungseigenschaft der Röntgenstrahlen-absorbierenden Schicht.

Als Alternative oder Ergänzung zu der Röntgenstrahlen-transparenten Schicht weist die Absorbermaske den mindestens einen sich in Breitenrichtung erstreckenden Röntgenstrahlen-absorbierenden Streifen auf. Wenn die Absorbermaske innerhalb einer Lage additiv aufgetragenes Röntgenstrahlen-transparentes Material und den Röntgenstrahlen-absorbierenden Streifen aufweist, ist eine solche Lage definitionsgemäß weiterhin eine Röntgenstrahlen-transparente Lage. In anderen Worten ändert die Unterbrechung des Röntgenstrahlen-transparenten Materials mit dem mindestens einen Röntgenstrahlen-absorbierenden Streifen nicht den Zweck dieser Lage, grundsätzlich die Röntgenstrahlen nicht vollständig zu absorbieren, sondern die Vielzahl von therapeutischen Röntgenstrahlen zu formen. Das gilt gleichermaßen, wenn innerhalb einer solche Lage gar kein Röntgenstrahlen-transparentes Material angeordnet ist, sondern der mindestens eine Röntgenstrahlen-absorbierende Streifen in einer Art Abstandshalter sicherstellt, dass die an den mindestens einen Streifen angrenzenden Lagen eine Öffnung, insbesondere einen Hohlraum umschließen, welcher materialfrei ist.

Je Lage kann nur ein Röntgenstrahlen-absorbierender Streifen oder mehrere Röntgenstrahlen-absorbierende Streifen angeordnet sein. Der mindestens eine Röntgenstrahlen-absorbierende Streifen bedeckt minimal 50%, beispielsweise mehr als 70% die Röntgenstrahlen-transparente Lage. Der mindestens eine Röntgenstrahlen-absorbierende Streifen kann in die ansonsten Röntgenstrahlen-transparenten Schichten integriert sein. Dieser Streifen kann aus dem gleichen Material bestehen wie die Röntgenstrahlen-absorbierende Schicht oder aus einem anderen Material mit hoher Röntgenabsorption. Der Streifen kann beispielsweise durch selektives Auftragen des absorbierenden Materials während des additiven Herstellungsprozesses erzeugt werden. Der mindestens eine Röntgenstrahlen-absorbierende Streifen kann sich über die gesamte Breitenrichtung der Lage erstrecken oder nur über einen Teil davon.

Der Zweck dieser Röntgenstrahlen-absorbierenden Streifen kann darin bestehen, die therapeutischen Röntgenstrahlen in einer weiteren Dimension zu formen. Während die mehreren Röntgenstrahlen-absorbierenden Schichten hauptsächlich Röntgenstrahlung in einer Richtung reduzieren, kann der mindestens eine Röntgenstrahlen-absorbierende Streifen in den Röntgenstrahlen-transparenten Lagen Röntgenstrahlung in einer dazu orthogonalen Richtung reduzieren. Die Leistung der Absorbermaske kann durch diese Konfiguration in mehrfacher Hinsicht beeinflusst werden. Zum einen kann eine verbesserte therapeutische Röntgenstrahlungsformung in zwei Dimensionen erreicht werden, was zu einer höheren Qualität bei der Strahlentherapie führen kann. Zum anderen kann die Flexibilität in der Gestaltung der Absorbermaske erhöht werden, da die Dichte und/oder Anordnung des mindestens einen Röntgenstrahlen-absorbierenden Streifens an spezifische Anforderungen angepasst werden kann. Insbesondere kann dadurch die Absorbermaske die Gitterform aufweisen. Die Ausdehnung in Breitenrichtung sowie Längenrichtung und der Abstand des mindestens einen Röntgenstrahlen-absorbierenden Streifens kann variiert werden, um die gewünschten Absorptionseigenschaften zu erzielen. Beispielsweise können die Streifen eine Ausdehnung in Längsrichtung der Lage von 50 bis 500 µm aufweisen und in Abständen von 1 bis 10 mm angeordnet sein. Die Dicke des Streifens kann der Dicke der Lage entsprechen, in die sie integriert ist, oder davon abweichen.

Die Integration des mindestens einen Röntgenstrahlen-absorbierenden Streifens in oder als eine Röntgenstrahlen-transparente Schicht kann durch verschiedene Methoden erfolgen. Bei additiven Fertigungsverfahren kann das Röntgenstrahlen-absorbierende Material gezielt an den gewünschten Positionen, beispielsweise im Wechsel mit Röntgenstrahlen-transparenten Material oder im Wechsel mit einem oder mehreren Hohlräumen, aufgetragen werden. Alternativ können vorgefertigte absorbierende Streifen in die transparenten Schichten eingebettet werden.

Beim übereinander Auftragen der Lagen erfolgt die additive Fertigung insbesondere derart, dass Material, welches in einer Lage aufgetragen wird, unter welcher sich ein Hohlraum bildet, nicht den Hohlraum auffüllt, sondern überbrückt. In anderen Worten erfolgt die Fertigung der Lagen vorzugsweise derart, dass die durch die additiv gefertigten Streifen-begrenzten Hohlräume materialfrei bleiben. Das Material einer Lage füllt insbesondere nicht einen Hohlraum einer anderen Lage auf. Ein Hohlraum ist insbesondere nach der Fertigung der Absorbermaske weiterhin materialfrei.

Die beschriebene Variante weist also zusammengefasst folgende Vorteile auf: Diese Konfiguration kann eine Optimierung der Absorbermaske für spezifische Anwendungen bei der Strahlentherapie ermöglichen. Durch Anpassung der Streifengeometrie und -anordnung kann die Absorbermaske an verschiedene Patienten angepasst werden. In die Lage aus Röntgenstrahlen-transparentem Material können durch Ansteuerung der Anlage zur additiven Herstellung einzelne Streifen aus Röntgenstrahlen-absorbierenden Material integriert werden. Auch wenn diese Streifen prozessbedingt in Längsrichtung eine größere Ausdehnung aufweisen können als die Lagendicke, insbesondere aufgrund der anisotropen minimalen Strukturgröße, können Absorbermasken in Gitterform gefertigt werden. Da die Lage und Anzahl der absorbierenden Streifen in den einzelnen Röntgenstrahlen-transparenten Lagen beliebig vorgegeben werden kann, kann eine Absorbermaske, deren Geometrie optimal auf die geplanten Einsatzmöglichkeiten angepasst ist, gefertigt werden.

Die Absorbermaske kann insgesamt eine Vielzahl an Schichten aufweisen. Typischerweise wechseln sich Röntgenstrahlen-transparente Schichten und die mehreren Röntgenstrahlen-absorbierenden Schichten insbesondere regelmäßig, vorzugsweise periodisch, in Wechselrichtung ab. Die Wechselrichtung der Schichten bezeichnet insbesondere die Richtung, in welcher sich die Röntgenstrahlen-transparente Lage und die mehreren Röntgenstrahlen-absorbierende Lagen abwechseln. Die Wechselrichtung der Schichten verläuft insbesondere in die Richtung, in welcher sich die Transmissionsraten der Lagen und/oder Materialmengen in Hinblick auf ganze Lagen und lediglich nur Streifen aus Röntgenstrahlen-absorbierendem Material abwechseln.

Die Stapelrichtung der Lagen bezieht sich insbesondere auf die Stapelrichtung des Herstellungsverfahrens, also die Richtung, in der die Lagen übereinander flächig aufgetragen werden. Die Stapelrichtung steht typischerweise senkrecht auf der Ebene der flächigen Lage. Die Stapelrichtung verläuft erfindungsgemäß im Wesentlichen parallel zur Oberseite bzw. Oberfläche der Absorbermaske. Also verläuft die Stapelrichtung insbesondere nicht senkrecht zur Oberseite bzw. Oberfläche der Absorbermaske. Erfindungsgemäß entspricht die Wechselrichtung der Schichten der Stapelrichtung der Lagen.

Der große Vorteil der Erfindung liegt also darin, dass die kritischen dünnen Strukturen über die Lagendicke gegeben werden. Besonders vorteilhaft ist, dass die benachbarten Lagen des Schichtstapels in Stapelrichtung sowie gleichzeitig in Wechselrichtung aufgetragen sind bzw. werden, denn die Fläche der einzelnen Lagen kann mit sehr breitem Materialauftrag, beispielsweise größer 100 µm oder im Bereich 0,1 mm bis 1 mm, aber in Dickenrichtung besonders dünn oder auch dick erzeugt werden. Dadurch können vorteilhafterweise äußerst dünne insbesondere Röntgenstrahlen-transparente Lagen und somit Röntgenstrahlen-transparente Schichten gefertigt werden. Alternativ oder zusätzlich reduziert sich die Fertigungszeit der einzelnen Lage gegenüber der herkömmlichen Praxis, die Lagen senkrecht zur Oberseite der Absorbermaske und somit senkrecht zur Wechselrichtung aufzubauen. Denn für eine Röntgenstrahlen-transparente Schicht ist es herkömmlicherweise insbesondere in üblichen 3D-Druck-Verfahren notwendig gewesen, diese Schicht in 5 oder mehr einzelne Raumpunkte je Lage aufzuteilen, was eine Fertigung der Absorbermaske mit Maßen von beispielsweise 400 mm x 400 mm oder 300 mm x 300 mm im Stand der Technik äußerst aufwändig macht.

Die beschriebene Absorbermaske kann insgesamt also mehrere technische Vorteile bieten. Durch die Verwendung des additiven Herstellungsverfahrens kann eine sehr präzise Kontrolle über die Lagendicken und -geometrien erreicht werden. Dies kann zu einer verbesserten Effizienz bei der Strahlentherapie führen. Weiterhin kann die Verwendung von alternativen Materialien anstelle von Blei die Umweltverträglichkeit der Absorbermaske verbessern. Die Möglichkeit, komplexe Strukturen zu fertigen, kann zudem eine Optimierung der Strahlentherapie für spezifische Anwendungen ermöglichen.

Eine Ausführungsform sieht vor, dass eine minimale Strukturgröße innerhalb einer Lage größer ist als eine minimale Dicke dieser Lage. Die minimale Dicke einer Lage kann die geringste Ausdehnung in Stapelrichtung darstellen, die für eine einzelne Lage fertigungstechnisch erreicht werden kann. Ein Beispiel für diese Konfiguration kann eine Lage sein, bei der die minimale Strukturgröße innerhalb der Lage 100 µm beträgt, während die minimale Dicke der Lage 20 µm oder 50 µm sein kann. Im Kern wird in dieser Ausführungsform ein anisotropes additives Herstellungsverfahren beschrieben, bei dem die Lagen sehr dünn, z.B. eine Lagendicke in der Größenordnung von 20 µm ist, während der Materialauftrag in den beiden zur Stapelrichtung orthogonalen Richtungen deutlich gröbere minimale Strukturgrößen größer als 20 µm, beispielsweise im Bereich von 50 µm bis 1000 µm aufweist. Die minimale Strukturgröße kann sich auf die kleinste laterale Abmessung beziehen, die innerhalb einer Lage der Absorbermaske hergestellt werden kann. Diese Größe kann durch die Auflösung des verwendeten additiven Herstellungsverfahrens bestimmt sein. Beispielsweise kann bei einem 3D-Druck-Verfahren die minimale Strukturgröße durch den Durchmesser des Querschnitts der Auftragungsdüse der Auftragseinheit der Anlage zur additiven Herstellung oder die Präzision der Positionierung der Auftragungseinheit beeinflusst werden. Die minimale Strukturgröße kann alternativ oder zusätzlich von Faktoren wie der Viskosität des verwendeten Materials, der Oberflächenspannung und/oder den Aushärtungseigenschaften abhängen. Beispielsweise kann bei einem Stereolithographie-Verfahren die Schichtdicke durch die Eindringtiefe des aushärtenden Lichts präzise gesteuert werden, während die laterale Auflösung durch den Fokusdurchmesser des Lasers begrenzt sein kann. Alternativ kann bei einem Pulverbett-Fusionsverfahren die Schichtdicke durch die Höhe der aufgetragenen Pulverschicht bestimmt werden, während die minimale Strukturgröße von der Korngröße des Pulvers und der Präzision des Energieeintrags abhängen kann.

Die Beziehung zwischen minimaler Strukturgröße und minimaler Dicke kann verschiedene Auswirkungen auf die Leistung der Absorbermaske haben. Eine größere minimale Strukturgröße im Vergleich zur minimalen Dicke kann zu einer verbesserten mechanischen Stabilität der Lage führen. Dies kann besonders wichtig für die Röntgenstrahlen-absorbierenden Schichten sein, da diese aus dichteren Materialien bestehen und daher anfälliger für strukturelle Schwächen sein können. Zudem kann diese Ausführungsform eine möglichst geringe Lagendicke bzw. Schichtdicke ermöglichen. Dies kann für die Optimierung der Absorbermaske von Vorteil sein.

Eine Ausführungsform sieht vor, dass eine Dicke einer der mehreren Röntgenstrahlen-absorbierenden Schichten größer ist als eine Dicke der Röntgenstrahlen-transparenten Schicht bzw. des mindestens einen Röntgenstrahlen-absorbierenden Streifens. Die Dicke einer der mehreren Röntgenstrahlen-transparenten Schichten kann weniger als 200 µm, insbesondere weniger als 100 µm, vorzugsweise weniger als 50 µm, besonders vorteilhafterweise weniger als 25 µm, betragen. Vorzugsweise kann eine dünnere Schicht die Absorption der primären Röntgenstrahlung minimieren, während gleichzeitig eine effektive Formung der therapeutischen Röntgenstrahlung erreicht wird. Beispiele für spezifische Dicken innerhalb des genannten Bereichs können 160 µm, 100 µm, 50 µm, 40 µm, 25 µm oder 20 µm sein. Eine Dicke von 100 µm kann beispielsweise einen guten Kompromiss zwischen Absorptionsfähigkeit und Gesamtdicke der Absorbermaske darstellen. Die Wahl der spezifischen Dicke kann von verschiedenen Faktoren abhängen, wie zum Beispiel dem verwendeten Absorptionsmaterial und den spezifischen Anforderungen der Strahlentherapieanwendung.

Eine Ausführungsform sieht vor, dass eine der mehreren Röntgenstrahlen-absorbierenden Schichten maximal eine Lage aufweist und/oder wobei eine Röntgenstrahlen-transparente Schicht maximal eine Lage aufweist. In diesem Fall beträgt insbesondere die Dicke der einen Lage weniger als 200 µm, insbesondere weniger als 100 µm. Diese Ausführungsform beschreibt unter anderem eine Absorbermaske mit einer vereinfachten Schichtstruktur, bei der jede der Schichten - die Röntgenstrahlen-transparente Schicht und die mehreren Röntgenstrahlen-absorbierenden Schichten aus maximal einer Lage besteht. Die Verwendung von maximal einer Lage pro Schicht kann den Herstellungsprozess der Absorbermaske vereinfachen. Bei einem additiven Herstellungsverfahren kann dies bedeuten, dass jede Schicht in einem einzigen Durchgang aufgetragen wird, was die Fertigungszeit und den Materialverbrauch reduzieren kann. Zudem kann diese Vorgehensweise die Präzision der Schichtdicken erhöhen, da Ungenauigkeiten, die durch das mehrfache Auftragen von Lagen entstehen können, vermieden werden. Da jede Schicht aus einer einzigen Lage besteht, kann die Zusammensetzung und Struktur des Materials innerhalb der Schicht homogener sein, was zu einer gleichmäßigeren Absorption oder Transmission der Röntgenstrahlung führen kann. Eine mögliche Anordnung der Lagen kann wie folgt aussehen: Eine einzelne Lage der Röntgenstrahlen-absorbierenden Schicht mit einer Dicke von 100 µm, gefolgt von einer einzelnen Lage der Röntgenstrahlen-transparenten Schicht mit einer Dicke von 20 µm, und abschließend eine einzelne Lage der Röntgenstrahlen-absorbierenden Schicht mit einer Dicke von 100 µm. Durch Variation der Dicke und Zusammensetzung der einzelnen Lagen kann die Maske für spezifische Anwendungen bei der Strahlentherapie optimiert werden, ohne die Komplexität des Herstellungsprozesses zu erhöhen.

Eine Ausführungsform sieht vor, dass ein Querschnitt einer Lage, insbesondere einer Lage der mehreren Röntgenstrahlen-absorbierenden Schichten, in Breitenrichtung trapezförmig ausgebildet ist. Ein trapezförmiger Querschnitt kann in diesem Zusammenhang als eine geometrische Form verstanden werden, bei der die obere und untere Kante der Lage parallel zueinander sowie parallel zur Stapelrichtung verlaufen, jedoch unterschiedliche Längen aufweisen. Die Seitenkanten der Lage können dabei schräg verlaufen und die obere mit der unteren Kante verbinden. Die Breitenrichtung steht auf der Stapelrichtung senkrecht sowie auf der Oberseite der Absorbermaske senkrecht. Die Trapezform ist insbesondere eine Keilstumpfform. Es ist denkbar, dass ein Querschnitt mehrerer Lagen oder aller Lagen in Breitenrichtung trapezförmig ausgebildet ist. Der Querschnitt zweier oder mehrerer Lagen kann verschieden sein, insbesondere mindestens einen unterschiedlichen Innenwinkel aufweisen, sprich nicht deckungsgleich sein. Beispielsweise können die insbesondere trapezförmigen Querschnitte unterschiedlich ausgerichtet, insbesondere geneigt, sein. Die Dickenvariation kann für verschiedene Lagen des Schichtstapels unterschiedlich gestaltet werden. Beispielsweise kann in einer Röntgenstrahlen-transparenten Lage eine andere Dickenvariation verwendet werden als in einer Röntgenstrahlen-absorbierenden Lage. Dies kann eine präzise Abstimmung der Absorptionseigenschaften der Absorbermaske ermöglichen.

Die trapezförmige Ausbildung des Querschnitts einer Lage kann durch verschiedene Fertigungsmethoden erreicht werden. Bei additiven Herstellungsverfahren kann beispielsweise die Auftragsmenge des Materials während des Auftragens einer Lage gezielt variiert werden, um die gewünschte Trapezform zu erzeugen. Insbesondere kann eine Dicke einer Lage des Schichtstapels mittels des additiven Herstellungsverfahrens in Breitenrichtung variiert werden. Bei einem Verfahren mit flüssigem oder pastösem Materialauftrag kann beispielsweise die Auftragsmenge des Materials entlang der Breitenrichtung der Lage gezielt verändert werden. Dies kann durch Anpassung des Materialflusses, der Bewegungsgeschwindigkeit der Auftragungseinheit oder einer Kombination beider Parameter erfolgen. Bei einem pulverbasierten additiven Herstellungsverfahren kann die Dickenvariation durch selektives Verfestigen unterschiedlicher Mengen von Pulvermaterial in verschiedenen Bereichen der Lage erreicht werden. Dies kann durch Variation der Energiemenge, die zum Verfestigen des Pulvers verwendet wird, oder durch mehrfaches Überfahren bestimmter Bereiche realisiert werden.

Die Neigung der Seitenkanten des Trapezes kann variieren und an die spezifischen Anforderungen der Absorbermaske angepasst werden. Beispielsweise kann ein Neigungswinkel zwischen 0,001° und 45° gewählt werden. Ein trapezförmiger Querschnitt einer Lage kann verschiedene potenzielle Vorteile für die Leistung der Absorbermaske bieten. Zum einen kann diese Form zu einer Fokussierung der Absorbermaske beitragen. Die schrägen Seitenkanten können als eine Art Führung für die Röntgenstrahlen dienen und unerwünschte Röntgenstrahlung reduzieren. Wesentlich vorteilhaft ist, wenn die Absorbermaske auf den Zielbereich im Gewebe fokussiert ist. Dies bedeutet insbesondere, dass Schichten im Zentrum der Absorbermaske senkrecht zur Oberfläche stehen, während Schichten mit zunehmender Entfernung zum Zentrum immer stärker in Richtung Zielbereich geneigt sind. Dies kann zu einer erhöhten Qualität bei der Strahlentherapie führen. Die trapezförmige Ausbildung des Querschnitts kann zur Optimierung der Absorptionseigenschaften der Absorbermaske beitragen. Durch die Variation der Dicke innerhalb einer insbesondere Röntgenstrahlen-absorbierenden Lage kann eine graduelle Änderung der Absorptionseigenschaften erreicht werden. Dies kann besonders nützlich sein, um die Maske an spezifische Strahlentherapiemodalitäten anzupassen. Alternativ oder zusätzlich kann die trapezförmige Form einer Lage dazu beitragen, Reflexionen und Streuungen an den Grenzflächen zwischen den Lagen zu reduzieren. Die Verwendung einer Lage mit trapezförmigem Querschnitt kann insbesondere die Flexibilität in der Gestaltung der Absorbermaske erhöhen. Durch Variation der Trapezform in verschiedenen Lagen können komplexe dreidimensionale Strukturen innerhalb der Absorbermaske erzeugt werden, die an spezifische Anforderungen der Strahlentherapie angepasst sind.

Eine Ausführungsform sieht vor, dass die Röntgenstrahlen-transparente Schichten aus einer ersten Materialzusammensetzung und die mehreren Röntgenstrahlen-absorbierenden Schichten aus einer zweiten Materialzusammensetzung ausgebildet sind, wobei die erste Materialzusammensetzung und die zweite Materialzusammensetzung bezüglich eines Anteils eines Materials voneinander abweichend ausgebildet sind. Die erste Materialzusammensetzung und die zweite Materialzusammensetzung können insbesondere bezüglich eines Anteils ausschließlich eines Materials oder mehrerer Materialien voneinander abweichend ausgebildet sein. Ein Anteil kann zwischen 0% und 100% liegen. Anteile weichen definitionsgemäß in dieser Ausführungsform voneinander ab, wenn aufgrund der Abweichung die Röntgenstrahlungsabschwächungseigenschaft der ersten Materialzusammensetzung sich von der Röntgenstrahlungsabschwächungseigenschaft der zweiten Materialzusammensetzung derart unterscheidet, dass die erste Materialzusammensetzung Röntgenstrahlen-transparent und dass die zweite Materialzusammensetzung Röntgenstrahlen-absorbierend ist. Eine Materialzusammensetzung umfasst ein einzelnes Material oder eine spezifische Kombination von Materialien, die zusammen die Eigenschaften einer Schicht bestimmen. Insbesondere die erste Materialzusammensetzung kann nur ein Material umfassen, während die zweite Materialzusammensetzung zwei oder mehr Materialien umfassen kann. Sobald eine Materialzusammensetzung ein Material der anderen Materialzusammensetzung nicht umfasst, unterscheiden sich die beiden Materialzusammensetzungen, denn der Anteil des einen Materials ist gleich null bei der einen Materialzusammensetzung und ungleich null bei der anderen Materialzusammensetzung. Die Materialzusammensetzung kann verschiedene Materialien, beispielsweise Elemente, Verbindungen oder Strukturen in unterschiedlichen Anteilen umfassen. Die erste Materialzusammensetzung der Röntgenstrahlen-transparenten Schicht kann beispielsweise aus leichten Materialien mit niedriger Ordnungszahl bestehen. Mögliche Materialien hierfür können Kunststoffe wie Polymere, Polyethylen, Epoxidharz, Polypropylen oder Acrylglas sein. Zusätzlich können diese Materialien mit Additiven oder Füllstoffen versetzt sein, um spezifische Eigenschaften zu erzielen. Die zweite Materialzusammensetzung der Röntgenstrahlen-absorbierenden Schicht kann Materialien mit hoher Ordnungszahl aufweisen. Solche Materialien können Metalle wie Blei, Tantal, Wolfram, Rhenium, Osmium, Iridium, Bismut, Platin, Thallium, Quecksilber oder Gold sein, die in Form von feinen Pulvern oder Nanopartikeln in eine Träger-Matrix, beispielsweise aus Kunststoff, eingebettet sein können.

Eine Ausführungsform sieht vor, dass die mindestens eine Röntgenstrahlen-transparente Schicht aus einer ersten Materialzusammensetzung ausgebildet ist, wobei die erste Materialzusammensetzung Glashohlkugeln und/oder ein Aerogel aufweist. Glashohlkugeln, auch als Mikrohohlglaskugeln oder "Microballons" oder Glasmikrosphären bezeichnet, sind mikroskopisch kleine, hohle Kugeln aus Glas. Diese Kugeln haben typischerweise einen Durchmesser von wenigen Mikrometern, beispielsweise 80 µm, bis zu einigen hundert Mikrometern und bestehen insbesondere aus einer dünnen Glasschale, die ein Gasvolumen oder Vakuum umschließt. Aufgrund ihrer Struktur weisen Glashohlkugeln eine sehr geringe Dichte auf, typischerweise im Bereich von 0,125 bis 0,6 g/cm³. Diese geringe Dichte in Kombination mit den Eigenschaften des Glases macht Glashohlkugeln zu einem hervorragenden Material für Röntgenstrahlen-transparente Schichten. Technisch lassen sich durch Mischen der Glashohlkugeln mit einem Epoxidharz oder mit einem flüssigen, beispielsweise thermoplastischen, Kunststoff ein Material mit sehr geringer Dichte herstellen. Bei hohen Glashohlkugeln-Konzentrationen ergibt sich eine Art Schaum mit einer Dichte von unter der von Wasser (1,0 g/cm3). Vorteilhafterweise werden die Glashohlkugeln für die Absorbermaskenfertigung zur Dichte- bzw. Absorptionsreduktion derart eingesetzt: z.B. die Glashohlkugeln sind eingebettet in einen thermoplastischen Kunststoff oder ein Epoxidharz gemäß dieser Ausführungsform. Insgesamt ermöglicht die Verwendung der Glashohlkugeln eine sehr deutliche Reduktion der unerwünschten Absorption der primären Röntgenstrahlung in einer Röntgenstrahlen-transparenten Schicht. Insbesondere in Kunststoffe eingebettete Glashohlkugeln ermöglichen durch die erreichbare sehr geringe Dichte die deutliche Reduktion von Absorption von primärer Röntgenstrahlung. Diese Ausführungsform erlaubt es, Absorbermasken wesentlich effektiver und zuverlässiger zu fertigen.

Für die Herstellung von Schichten mit Glashohlkugeln kann eine Methode verwendet werden, bei der die Glashohlkugeln in eine Träger-Matrix, insbesondere aus einem Kunststoff eingebettet werden. Dies kann durch Mischen der Glashohlkugeln mit dem Kunststoff, insbesondere einem flüssigen Kunststoff, und durch anschließendes Aushärten erfolgen. Alternativ können die Glashohlkugeln auch in ein Pulverbett eingebracht und anschließend durch Sintern oder Schmelzen verbunden werden.

Alternativ oder zusätzlich lassen sich Glashohlkugeln-gefüllte Werkstoffe auch durch Aerogele mit noch geringer Dichte ergänzen oder ersetzen. Aerogele sind hochporöse Festkörper mit einer geringen Dichte. Sie bestehen zu einem Großteil aus Luft (oft über 95% des Volumens) und weisen eine netzartige Struktur aus miteinander verbundenen Nanopartikeln auf. Aerogele können aus verschiedenen Materialien hergestellt werden, wobei Silica-Aerogele die bekanntesten sind. Die geringe Dichte und die offene Struktur machen Aerogele ebenfalls zu einem ausgezeichneten Material für Röntgenstrahlen-transparente Schichten. Diese Aerogele stehen typischerweise in deutlich größeren Stärken von einigen mm zur Verfügung. Es ist denkbar, Aerogele in der additiven Fertigung, insbesondere beim 3D-Druck einzusetzen. Insbesondere Aerogel-Schichten im Bereich von 100 µm Dicke stellen eine Ergänzung oder Alternative zur Verwendung von Glashohlkugeln dar.

Bei der Verwendung von Aerogelen kann eine Sol-Gel-Methode angewendet werden, bei der zunächst ein Gel hergestellt und anschließend durch überkritische Trocknung das Lösungsmittel entfernt wird, ohne die Porenstruktur zu zerstören. Eine alternative Methode ist die Gefriertrocknung, bei der das Lösungsmittel durch Sublimation entfernt wird.

In beiden Fällen kann das additive Herstellungsverfahren angepasst werden, um die speziellen Eigenschaften dieser Materialien optimal zu nutzen.

Die Verwendung von Glashohlkugeln und/oder Aerogelen in der ersten Materialzusammensetzung kann die Röntgentransparenz der entsprechenden Schichten erheblich verbessern, also die Röntgenstrahlungsschwächungseigenschaft verringern zugunsten einer höheren Röntgenstrahlungstransmissionseigenschaft, was wiederum die Effizienz der Absorbermaske erhöhen kann. Dies liegt daran, dass beide Materialien einen hohen Anteil an Luft oder Gas enthalten, welche Röntgenstrahlen nahezu ungehindert passieren lassen. Gleichzeitig können diese Materialien die mechanische Stabilität und andere wichtige Eigenschaften der Schichten positiv beeinflussen. Insbesondere kann die geringe Dichte dieser Materialien zu einer Reduktion des Gesamtgewichts der Absorbermaske beitragen, was in bestimmten Anwendungen vorteilhaft sein kann. Beispielsweise können spezielle Auftragungsdüsen verwendet werden, die eine gleichmäßige Verteilung der Glashohlkugeln oder eine kontrollierte Abscheidung des Aerogels ermöglichen.

Eine Ausführungsform sieht vor, dass die mehreren Röntgenstrahlen-absorbierenden Schichten aus einer zweiten Materialzusammensetzung ausgebildet sind, wobei die zweite Materialzusammensetzung Blei, Tantal, Wolfram, Rhenium, Osmium, Iridium, Bismut, Platin, Thallium, Quecksilber oder Gold aufweist. Besonders vorteilhafterweise werden diese Elemente, also die genannten Metalle der 6. Periode, in die beschriebene Träger-Matrix eingebettet und in der erfindungsgemäßen Anlage zur additiven Fertigung eingesetzt. Hierdurch besteht vorzugsweise fast völlige Freiheit der geometrischen Gestaltung von Absorbermasken. Die zweite Materialzusammensetzung bildet insbesondere die mehreren Röntgenstrahlen-absorbierenden Schichten der Absorbermaske. Die Verwendung der vorgenannten Materialien mit hoher Ordnungszahl und hoher Dichte ist entscheidend für eine effektive Absorption von Röntgenstrahlung. Die genannten Elemente weisen diese Eigenschaften auf und können daher besonders geeignet für den Einsatz in der Röntgenstrahlen-absorbierenden Schicht sein.

Blei kann aufgrund seiner hohen Dichte von etwa 11,3 g/cm³ und seiner guten Absorptionseigenschaften für Röntgenstrahlung verwendet werden. Blei als Element der 6. Periode im Periodensystem weist eine hohe für Röntgenstrahlenabsorption relevante Kernladungszahl auf. Aufgrund der zunehmend niedrigen Kernladungszahlen kommen als Ersatz für Blei insbesondere folgende stabile Elemente der 6. Periode in Frage, unter gewissen Bedingungen auch Thallium und Quecksilber trotz deren Toxizität. Insbesondere kommen damit die bevorzugten Elemente Tantal, Wolfram, Rhenium, Osmium, Iridium, Bismut, Platin oder Gold als Ersatz für Blei in Frage. Neben der Ordnungszahl der bevorzugten Elemente ist deren Dichte sehr relevant. Bei gegebener Materialstärke für Blei (beispielsweise 20 µm), lässt sich die benötigte Materialstärke, für die bevorzugten Elemente, berechnen. Tantal weist eine höhere Dichte von etwa 16,7 g/cm^3 als Blei auf. Wolfram kann mit einer Dichte von etwa 19,3 g/cm^3 eine hervorragende Absorptionsfähigkeit für Röntgenstrahlung bieten. Rhenium kann mit einer Dichte von etwa 21,0 g/cm^3 eine sehr hohe Absorptionsfähigkeit aufweisen. Osmium weist die höchste Dichte von etwa 22,6 g/cm3 unter den genannten Elementen auf. Iridium kann mit einer Dichte von etwa 22,6 g/cm^3 ebenfalls eine sehr hohe Absorptionsfähigkeit aufweisen. Platin kann mit einer Dichte von etwa 21,5 g/cm^3 eine ausgezeichnete Absorptionsfähigkeit für Röntgenstrahlung bieten. Gold kann mit einer Dichte von etwa 19,3 g/cm^3 eine sehr gute Absorptionsfähigkeit aufweisen. Quecksilber weist mit 13,6 g/cm^3 und Thallium mit 11,7 g/cm^3 eine höhere Dichte als Blei auf, während die Dichte von Bismut mit 9,8 g/cm^3 niedriger ist.

Es ergibt sich, dass alle oben genannten bevorzugten Elemente vorteilhafter als Blei einsetzbar sind, denn es ergeben sich bei gleichzeitiger Abschirmwirkung geringere Materialstärken. Von den genannten Elementen kommt vor allem Wolfram aufgrund seines relativ geringen Preises für einen Einsatz in Betracht. Speziell die Elemente Osmium, Gold und Platin können aufgrund ihrer vergleichsweise hohen Preise aus kommerziellen Gründen für die Verwendung in Absorbermasken ausscheiden.

Die Einbettung dieser Materialien in die Absorbermaske kann auf verschiedene Weise erfolgen. Eine Möglichkeit kann die Einbettung von feinen Pulvern oder Nanopartikeln dieser Materialien in eine Träger-Matrix oder Legierungen mit solchen Materialien sein.

Die Verwendung dieser Materialien in der zweiten Materialzusammensetzung kann verschiedene Vorteile bieten. Zum einen kann eine hohe Absorptionseffizienz erreicht werden, was zu einer effektiven Formung der Vielzahl an therapeutischen Röntgenstrahlen führen kann. Zum anderen kann durch die Wahl des Materials und der Verarbeitungsform die Dicke der absorbierenden Schicht optimiert werden, was zu einer Verringerung der Gesamtdicke der Absorbermaske führen kann. Darüber hinaus kann die Verwendung von alternativen Materialien zu Blei die Umweltverträglichkeit der Absorbermaske verbessern.

Mit bekannten Verfahren können viele Metalle chemisch bzw. elektrochemisch aus wässeriger Lösung abgeschieden werden. Bei der konkreten Wahl des Elementes, ist es wichtig, dass sich das Element industriell abscheiden lässt. Hier ist insbesondere Gold trotz des hohen Preises eine gute Alternative, da entsprechende Verfahren, Gold abzuscheiden in der elektrotechnischen Industrie zur Veredelung von Kontaktflächen gut etabliert sind. Elektrochemisch können aus wässeriger Lösung nur Elemente der 6. Gruppe von Rhenium bis Bismut abgeschieden werden. Wolfram lässt aufgrund seiner chemischen Beschaffenheit nicht aus wässriger Lösung abscheiden. Rhenium kommt erfindungsgemäß für eine elektrolytische Abscheidung in Frage und hat auch den niedrigsten Preis der oben genannten Metalle. Wolfram lässt sich jedoch mit neu bekannt gewordenen Verfahren (siehe Dominik Höhlich et. al.: Simultaneous Electrodeposition of Silver and Tungsten from [EMIm]Cl:AlCl3 Ionic Liquids outside the Glove Box; Coatings 2020, 10(6), 553; https://doi.org/10.3390/coatings10060553, DOI: 10.7395/2020/Hoehlich und die Patentschrift DE 10 2014 118 593 A1) zusammen mit Nickel oder Silber aus ionischer (nicht-wässeriger) Lösung abscheiden. Insbesondere ist eine entsprechende Abscheidung von Wolfram oder Wolfram in Verbindung mit einem anderen Metall möglich.

Wolfram kommt als relativ preiswertes Element in Frage. Da auch Tantal als Material ungiftig ist und es noch eine leicht bessere Röntgenstrahlungsschwächungseigenschaft als Blei aufweist, kann es auch als Material für eine Röntgenstrahlen-absorbierende Schicht vorgesehen werden. Insbesondere können Wolfram-Rhenium-Legierungen eingesetzt werden. Vorteil dieser Legierungen ist, dass das Mischungsverhältnis der beiden Elemente typischerweise keine Rolle bei der Röntgenstrahlungsschwächungseigenschaft spielt, denn diese ist für beide Elemente fast identisch. Eine weitere Möglichkeit stellen gut formbare Legierungen aus Wolfram mit beispielsweise Kupfer, Nickel und/oder Eisen dar. Da diese Legierungen technisch mit typischen Wolframgehalten von 90% bis 95% angeboten werden, führt die geringere Röntgenstrahlungsschwächungseigenschaft der beigemischten leichten Elemente, z.B. hat Kupfer auf das Volumen bezogen etwa 9% des Absorptionsvermögens von Blei, typischerweise nur zu einer geringen Zunahme der notwendigen Dicke der Röntgenstrahlen-absorbierenden Schichten.

Alternativ zu den Legierungen mit den bevorzugten Elementen können Mischungen von Pulvern der bevorzugten Elemente mit anderen Bindersubstanzen zur Herstellung der Röntgenstrahlen-absorbierenden Schichten verwendet werden. Als Binder oder Matrix-Material kommen sowohl Metalle wie insbesondere Silber oder Zinn in Frage, als auch Kunststoffe. Die Einbettung von Wolframpulver in Zinn ist bekannt aus DE 60 2004 000 309 T2. Alternativ eignen sich insbesondere Kunststoffe als Material zur Einbettung von Wolframpulver. Es sind kommerziell Materialien mit einem Wolframpulver-Gehalt von über 90% in einer Träger-Matrix aus Polyethylen verfügbar. Diese Materialien weisen Dichten von bis zu 15g/cm^3 auf.

Eine Ausführungsform sieht vor, dass die erste Materialzusammensetzung eine Kunststoff-Matrix aufweist und die zweite Materialzusammensetzung die gleiche Kunststoff-Matrix aufweist. Die Kunststoff-Matrix ist insbesondere eine Träger-Matrix aus Kunststoff. Dass beide Materialzusammensetzungen die gleiche Kunststoff-Matrix aufweisen, bedeutet nicht, dass die beiden Materialzusammensetzungen identisch sind. Die beiden Materialzusammensetzungen können zusätzlich jeweils ein Material aufweisen, welches in der jeweils anderen Materialzusammensetzung nicht enthalten ist. Es ist denkbar, dass die erste Materialzusammensetzung aus der Kunststoff-Matrix besteht und die zweite Materialzusammensetzung einen Anteil größer null an einem Röntgenstrahlen-absorbierenden Metall, wie z.B. den zuvor genannten Materialien, aufweist, welches in die gleiche Kunststoff-Matrix eingebettet ist. Die Kunststoff-Matrix bezeichnet ein polymeres Material, das als Grundstruktur oder Bindemittel für andere Materialien dient. In der Absorbermaske dieser Ausführungsform wird diese Träger-Matrix sowohl in einer Röntgenstrahlen-transparenten Schicht als auch in den mehreren Röntgenstrahlen-absorbierenden Schichten verwendet. Insbesondere für eine Röntgenstrahlen-transparente Schicht kann die Kunststoff-Matrix aus einem Material mit niedriger Ordnungszahl bestehen. Diese Materialien weisen eine geringe Absorption von Röntgenstrahlung auf und können daher eine hohe Transmission der primären Röntgenstrahlung ermöglichen. In den mehreren Röntgenstrahlen-absorbierenden Schichten wird in dieser Ausführungsform die gleiche Kunststoff-Matrix verwendet, jedoch insbesondere mit Zusätzen von Materialien mit hoher Ordnungszahl. Diese Zusätze können insbesondere in Form von feinen Pulvern oder Nanopartikeln in die Matrix eingebettet werden. Vorzugsweise können Wolfram- oder Tantalpulver in die Kunststoff-Matrix eingearbeitet werden, um die Röntgenabsorption zu erhöhen. Die Verwendung der gleichen Kunststoff-Matrix in beiden Materialzusammensetzungen kann mehrere Vorteile bieten. Zunächst kann eine verbesserte Haftung zwischen den Schichten erreicht werden, da die chemische Kompatibilität zwischen den Materialien erhöht wird. Dies kann zu einer verbesserten strukturellen Integrität der gesamten Absorbermaske führen. Weiterhin kann die Verwendung der gleichen Matrix die Herstellung der Absorbermaske vereinfachen. Bei additiven Fertigungsverfahren kann beispielsweise das gleiche Grundmaterial für alle Schichten verwendet werden, wobei lediglich ein Zusatz oder die Zusätze für die Röntgenstrahlen-absorbierende Schicht variiert werden. Dies kann den Fertigungsprozess effizienter gestalten und mögliche Kompatibilitätsprobleme zwischen verschiedenen Matrixmaterialien vermeiden. Zudem kann die Verwendung einer einheitlichen Matrix zu einer gleichmäßigeren thermischen Ausdehnung der verschiedenen Schichten führen. Dies kann thermische Spannungen und mögliche Verformungen der Absorbermaske bei Temperaturänderungen reduzieren. Die Wahl der spezifischen Kunststoff-Matrix kann von verschiedenen Faktoren abhängen, wie beispielsweise der gewünschten mechanischen Stabilität, der Verarbeitbarkeit im additiven Fertigungsprozess und der Kompatibilität mit den verwendeten Zusatzmaterialien für die absorbierende Schicht.

Alternativ ist es denkbar, dass die erste Materialzusammensetzung und die zweite Materialzusammensetzung verschiedene Kunststoff-Matrizen aufweisen. Insbesondere können sich die beiden Kunststoff-Matrizen in der Art des jeweiligen Kunststoffs, z.B. PET und PP, unterscheiden.

Idealerweise weist die Maschine mindestens zwei Auftragsdüsen auf, mit der unterschiedliche Materialien aufgetragen werden: Ein möglichst stark Röntgenstrahlen-absorbierendes Material mit einer der beiden Auftragsdüsen und ein möglichst Röntgenstrahlen-transparentes Material mit der anderen der beiden Auftragsdüsen. Grundsätzlich ist es denkbar, dass mit beiden Auftragsdüsen gleichzeitig, nacheinander und Lage für Lage abwechselnd entweder Röntgenstrahlen-absorbierendes Material oder Röntgenstrahlen-transparentes Material aufgetragen wird.

Durch einen abwechselnden Lagenaufbau der beiden Materialien entsteht die Absorbermaske. Als stark Röntgenstrahlen-absorbierendes Material kommt vorzugsweise eine schmelzbare Matrix, die zu einem hohen Anteil mit feinem Wolframpulver oder anderen Pulver von Elementen der 6. Periode des Periodensystems der Elemente gefüllt ist, in Frage. Röntgenstrahlen-absorbierend bedeutet insbesondere möglichst Röntgenstrahlen-dicht im Vergleich zu Röntgenstrahlen-transparent.

Eine Ausführungsform sieht vor, dass ein Abstand innerhalb einer Lage zwischen Röntgenstrahlen-absorbierenden Streifen kleiner gleich 10 mm, vorzugsweise kleiner gleich 1 mm, beträgt. Der Abstand bezieht sich auf die Längsrichtung der Lage. Alternativ oder zusätzlich kann ein Abstand innerhalb einer Lage zwischen Röntgenstrahlen-absorbierenden Streifen größer gleich 0,01 mm, vorzugsweise größer gleich 0,1 mm, betragen. Insbesondere kann ein Verhältnis innerhalb einer Lage zwischen einer Länge eines Röntgenstrahlen-absorbierende Streifen und einem benachbarten Abstand größer gleich 1, vorzugsweise größer gleich 2, betragen. Der Abstand innerhalb einer Lage zwischen Röntgenstrahlen-absorbierenden Streifen kann mit Röntgenstrahlen-transparenten Material befüllt oder materialfrei sein.

Eine Ausführungsform sieht vor, dass sich Röntgenstrahlen-absorbierende Streifen von benachbarten Lagen zu einer regelmäßigen Struktur zusammensetzen. Eine regelmäßige Struktur bezeichnet in diesem Zusammenhang eine geometrische Form mit N Ecken und N Seiten, die durch die Anordnung der Röntgenstrahlen-absorbierenden Streifen in benachbarten Lagen gebildet wird. Insbesondere ist die regelmäßige Struktur ein Rechteck oder Quadrat. Weitere Beispiele für regelmäßige Strukturen können Dreiecke (N=3), Fünfecke (N=5), Sechsecke (N=6) oder komplexere Polygone mit einer höheren Anzahl von Ecken sein. Ein Absorbermaske dieser Ausführungsform weist eine Gitterform auf. Die spezifische Wahl von N kann von den gewünschten Absorptionseigenschaften und der beabsichtigten Anwendung der Absorbermaske abhängen. Die Bildung dieser regelmäßigen Strukturen kann durch eine präzise Anordnung der Röntgenstrahlen-absorbierenden Streifen in aufeinanderfolgenden Lagen erreicht werden. Beispielsweise können in einer ersten Lage Streifen an einer bestimmten Position in Bezug auf die Längenausdehnung angeordnet sein, während in der darüberliegenden Lage die Streifen an einer dazu versetzten oder an der gleichen Position angeordnet ist. Durch die Überlagerung dieser Streifen können sich die regelmäßigen Strukturen ergeben.

Diese Ausführungsform kann die Leistung der Absorbermaske in mehrfacher Hinsicht beeinflussen. Zum einen kann eine verbesserte Formung der Vielzahl an therapeutischen Röntgenstrahlen in verschiedenen Richtungen erreicht werden, da die regelmäßigen Strukturen Röntgenstrahlen aus unterschiedlichen Winkeln absorbieren können. Zum anderen kann diese Anordnung zu einer gleichmäßigeren Verteilung der Absorption über die Fläche der Absorbermaske führen.

Die Herstellung einer solchen Absorbermaske mit regelmäßigen Strukturen kann durch additive Fertigungsverfahren realisiert werden. Bei diesen Verfahren können die Röntgenstrahlen-absorbierenden Streifen in jeder Lage präzise positioniert werden, um die gewünschten regelmäßigen Strukturen zu erzeugen. Diese Methoden können eine hohe Präzision bei der Erzeugung der regelmäßigen Strukturen ermöglichen. Die Größe der N-eckigen Strukturen kann variiert werden, um die Absorptionseigenschaften der Absorbermaske an spezifische Anforderungen anzupassen. Kleinere Strukturen können eine feinere Kontrolle der geformten therapeutischen Röntgenstrahlen ermöglichen, während größere Strukturen möglicherweise einfacher zu fertigen sind und insgesamt weniger Röntgenstrahlenabsorption verursachen.

Eine Ausführungsform sieht vor, dass der Schichtstapel in Abhängigkeit von einer Kontur eines Patienten derart gefertigt ist, dass eine Seite der Absorbermaske im Wesentlichen das Negativ der Kontur des Patienten aufweist. Die Seite, welche das Negativ aufweist, ist insbesondere eine konturierte Seite der Absorbermaske. Dass die konturierte Seite das Negativ aufweist, bedeutet insbesondere, dass die Oberfläche der Seite so konturiert ist, dass sie zur Kontur des Patienten passt. Die konturierte Seite ist beispielsweise die Oberseite der Absorbermaske. Die Kontur des Patienten ist insbesondere von einer Oberfläche, beispielsweise der Haut des Patienten, vorgegeben. Beispielsweise kann eine Abbildung die Kontur des Patienten zeigen, welche bei der Fertigung des Schichtstapels berücksichtigt wird. Die Abbildung kann eine Fotographie oder ein 3D-Bild oder ein 3D-Muster oder eine andere dreidimensionale Darstellung sein. Die Kontur des Patienten kann in Pixel oder in Vektor- oder Polygon-Daten vorliegen. Die Absorbermaske weist typischerweise eine Ausdehnung auf, welche klein ist im Vergleich zum Patienten. Die Absorbermaske ist üblicherweise auf diejenige Größenordnung beschränkt, in welcher der Zielbereich im Gewebe liegt. Somit hängt der Schichtstapel nicht von der Kontur des gesamten Patienten ab, sondern nur von einem üblicherweise geringen Anteil des Patienten. Die konturierte Seite der Absorbermaske ist vorzugsweise passgenau auf die Kontur des Patienten ausgebildet. Beispielsweise kann der Zielbereich im Gewebe des Oberarms eines Patienten liegen. In diesem Fall ist die konturierte Seite des Schichtstapels derart geformt, dass die Absorbermaske passgenau auf dem Oberarm aufliegen kann. Passgenau bedeutet insbesondere lückenlos. Diese Ausführungsform ist insbesondere vorteilhaft, weil die Absorbermaske patientenindividuell gefertigt wird. Dadurch kann vorzugsweise die Qualität der Strahlentherapie erhöht werden. Weiterhin kann die patientenindividuelle Absorbermaske eine aufwändige Vorrichtung zur Ausrichtung und/oder der Fixierung der Absorbermaske relativ zum Patienten überflüssig machen.

Eine erfindungsgemäße Anordnung für eine Strahlentherapie eines Patienten weist
- eine erfindungsgemäße Absorbermaske,
- eine therapeutische Röntgenstrahlenquelle und
- einen Therapiebereich auf,
- wobei die Absorbermaske zwischen dem Therapiebereich und der therapeutischen Röntgenstrahlenquelle angeordnet und derart ausgerichtet ist, dass die Stapelrichtung des Schichtstapels im Wesentlichen senkrecht zur Röntgenstrahlung der therapeutischen Röntgenstrahlenquelle ist.

Eine Röntgenstrahlenquelle kann eine Vorrichtung sein, die Röntgenstrahlung erzeugt. Die Röntgenstrahlenquelle kann dazu ausgebildet sein, Röntgenstrahlung mit einer bestimmten Energie oder einem bestimmten Energiespektrum zu erzeugen. Die Röntgenstrahlenquelle kann insbesondere ein Röntgenstrahler oder ein Beschleunigersystem sein. Der Röntgenstrahler umfasst insbesondere ein evakuiertes Gehäuse, in welchem insbesondere eine Anode und eine Kathode angeordnet sind. Die Kathode umfasst insbesondere einen Elektronenemitter, von welchem freie Elektronen in Richtung der Anode mittels einer Hochspannung oder Hochfrequenzpulsen beschleunigt werden können. Beim Auftreffen der beschleunigten Elektronen auf der Anode wird insbesondere die Röntgenstrahlung generiert. Der Elektronenemitter kann insbesondere ein kalter, beispielsweise ein Feldeffektemitter, oder ein thermionischer, beispielsweise ein Wendel- oder Blechemitter sein. Die beschriebene Methode ist jedoch nicht auf die exemplarisch genannten Verfahren und Vorrichtung der Röntgenstrahlungserzeugung beschränkt. Es sind auch beispielsweise Röntgenstrahlenquellen auf Basis der "inversen Comptonstreuung" bekannt. Dabei wird Röntgenstrahlung durch die Wechselwirkung von hochenergetischen Elektronen und sehr intensiven Laserstrahlen erzeugt. Weiterhin sind auch solche Röntgenstrahlenquellen bekannt, bei der Anoden aus flüssigen oder pulverförmigen Metallen zum Einsatz kommen.

Ein Therapiebereich kann ein Raum oder Volumen sein, in dem der zu therapierende Patient platziert werden kann. Der Therapiebereich ist insbesondere ein Behandlungsbereich. Die Anordnung der Komponenten kann so gestaltet sein, dass die Röntgenstrahlenquelle Röntgenstrahlung emittiert, die im Therapiebereich auf den Patienten zur Strahlentherapie trifft. Die Absorbermaske kann zwischen dem Therapiebereich und der Röntgenstrahlenquelle positioniert sein, um die Vielzahl von therapeutischen Röntgenstrahlen insbesondere für die Strahlentherapie des Patienten zu formen.

Da die erfindungsgemäße Anordnung die erfindungsgemäße Absorbermaske aufweist, teilt sie die zuvor beschriebenen Vorteile. Die Ausrichtung der Absorbermaske mit der Stapelrichtung des Schichtstapels im Wesentlichen senkrecht zur Röntgenstrahlung kann dazu dienen, die Qualität der Strahlentherapie zu optimieren. Die beschriebene Anordnung für eine Strahlentherapie kann zu einer Minimierung der Absorption der primären Röntgenstrahlung führen.

Die erfindungsgemäße Anordnung kann insbesondere für eine Strahlentherapie geeignet sein. Eine Anwendung der Strahlentherapie ist insbesondere eine Bestrahlung mit sehr inhomogenen Feldern, z.B. die "Spatially Fractionated Radio Therapy".

Eine Strahlentherapieanlage kann die erfindungsgemäße Anordnung sowie beispielsweise mindestens einen Steuerrechner umfassen, welcher die Röntgenstrahlenquelle für die Strahlentherapie ansteuern kann. Die Strahlentherapieanlage kann insbesondere für die Strahlentherapie ausgebildet sein.

Eine erfindungsgemäße Anlage zur additiven Herstellung einer Absorbermaske zum Formen einer Vielzahl von therapeutischen Röntgenstrahlen, insbesondere für eine Strahlentherapie eines Patienten, weist eine Auftragungseinheit auf, welche dazu ausgebildet ist, das erfindungsgemäße Herstellungsverfahren durchzuführen.

Eine Auftragungseinheit kann eine Vorrichtung sein, die dazu ausgebildet ist, Material schichtweise aufzutragen, um eine dreidimensionale Struktur zu erzeugen. Die Auftragungseinheit kann verschiedene Komponenten umfassen, wie beispielsweise einen oder mehrere Auftragungsdüsen, Extruder oder andere Mechanismen zum präzisen Auftragen von Material. Die Auftragungseinheit kann dazu ausgebildet sein, verschiedene Materialien zu verarbeiten und aufzutragen. Diese Materialien können Röntgenstrahlen-transparente Materialien der ersten Materialzusammensetzung für die Röntgenstrahlen-transparente Schicht sowie Röntgenstrahlen-absorbierende Materialien der zweiten Materialzusammensetzung für die mehreren Röntgenstrahlen-absorbierenden Schichten umfassen. Die Auftragungseinheit kann über Mechanismen verfügen, um zwischen verschiedenen Materialien zu wechseln oder mehrere Materialien gleichzeitig aufzutragen. Die Anlage kann so konfiguriert sein, dass die Auftragungseinheit Lagen flächig aufeinander in Stapelrichtung zu einem Schichtstapel aufträgt. Dabei kann die Anlage sicherstellen, dass der Schichtstapel die mehreren Röntgenstrahlen-absorbierenden Schichten sowie zwischen den mehreren Röntgenstrahlen-absorbierenden Schichten mindestens eine Röntgenstrahlen-transparente Schicht und/oder mindestens einen sich in Breitenrichtung erstreckenden Röntgenstrahlen-absorbierenden Streifen aufweist, wobei jede dieser Schichten mindestens eine flächige Lage aufweist. Die Anlage kann Steuerungsmechanismen umfassen, die es ermöglichen, die Dicke der aufgetragenen Lagen präzise zu kontrollieren. Dies kann besonders wichtig sein für die Herstellung von Absorbermasken mit sehr dünnen absorbierenden Schichten. Die Anlage kann über Positionierungssysteme verfügen, die eine präzise Bewegung der Auftragungseinheit in drei Dimensionen ermöglichen. Dies kann eine genaue Kontrolle über die Geometrie und Struktur der hergestellten Absorbermaske ermöglichen. Die Anlage kann auch Systeme zur Überwachung und Qualitätskontrolle des Herstellungsprozesses umfassen. Diese können beispielsweise Sensoren zur Messung der Schichtdicke oder zur Überprüfung der Materialzusammensetzung beinhalten. Eine solche Anlage zur additiven Herstellung einer Absorbermaske kann eine hohe Flexibilität in der Herstellung ermöglichen. Die Geometrie und Zusammensetzung der Absorbermaske kann leicht angepasst werden, um verschiedene Anforderungen in der Strahlentherapie zu erfüllen. Zudem kann die additive Fertigung die Herstellung komplexer interner Strukturen ermöglichen, die mit konventionellen Fertigungsmethoden schwer zu realisieren wären. Zusätzlich kann die Anlage dazu ausgebildet sein, die Dicke einer Lage des Schichtstapels in Breitenrichtung zu variieren. Dies kann durch präzise Steuerung des Materialauftrags während des additiven Herstellungsprozesses erreicht werden, beispielsweise durch Anpassung des Materialflusses und/oder der Bewegungsgeschwindigkeit der Auftragungseinheit.

Eine Ausführungsform sieht vor, dass die Auftragungseinheit mindestens eine Auftragungsdüse mit einem ovalen oder rechteckigen Querschnitt aufweist. Der Querschnitt der Auftragungsdüse betrifft die Öffnung der Auftragungsdüse, aus welcher das Material austritt. Die Querschnitte mehrerer Auftragungsdüsen können sich in Form und/oder Größe unterscheiden. Eine Auftragungsdüse mit einem ovalen oder rechteckigen Querschnitt kann mehrere Vorteile für den Herstellungsprozess und die resultierende Struktur der Absorbermaske bieten. Im Gegensatz zu einer konventionellen runden Düse kann eine ovale oder rechteckige Düse eine breitere Materialbahn in einem einzelnen Durchgang auftragen. Dies kann die Fertigungszeit reduzieren und die Effizienz des Herstellungsprozesses erhöhen.

Der ovale oder rechteckige Querschnitt der Auftragungsdüse kann es ermöglichen, Materialschichten mit einer kontrollierten Breite und Dicke aufzutragen. Die Form der Düse kann so gewählt werden, dass die aufgetragene Materialbahn der gewünschten Geometrie der Absorbermaske entspricht. Beispielsweise kann eine rechteckige Düse mit einem Seitenverhältnis von 10:1 eine Materialbahn auftragen, die 10 mal so breit wie dick ist. Die Verwendung einer ovalen oder rechteckigen Auftragungsdüse kann auch zur Verbesserung der Oberflächenqualität der hergestellten Absorbermaske beitragen. Durch den breiteren Materialauftrag können Oberflächenunebenheiten, die durch das schichtweise Auftragen entstehen können, reduziert werden. Dies kann zu einer glatteren Oberfläche der Absorbermaske führen, was für die Leistung in der Strahlentherapie vorteilhaft sein kann. Zudem kann die Form der Auftragungsdüse an die spezifischen Anforderungen der verschiedenen Schichten der Absorbermaske angepasst werden. Für die Röntgenstrahlen-transparenten Lagen kann beispielsweise eine breitere Auftragungsdüse verwendet werden, um eine schnelle Abdeckung großer Flächen zu ermöglichen. Für die Röntgenstrahlen-absorbierenden Lagen kann eine schmalere Düse eingesetzt werden, um eine präzisere Kontrolle über die Schichtdicke zu gewährleisten.

Die ovale oder rechteckige Form der Auftragungsdüse kann auch dazu beitragen, die innere Struktur der Absorbermaske zu optimieren. Durch gezielte Ausrichtung der Auftragungsdüse während des Auftragens können Materialstränge erzeugt werden, die in einer bestimmten Richtung orientiert sind. Dies kann genutzt werden, um die Absorptionseigenschaften der Maske in verschiedenen Richtungen zu steuern. Eine Anlage zur additiven Herstellung einer Absorbermaske mit einer Auftragungseinheit, die mindestens eine Auftragungsdüse mit ovalem oder rechteckigem Querschnitt aufweist, kann eine verbesserte Kontrolle über den Herstellungsprozess und die resultierende Maskenstruktur ermöglichen. Dies kann zu einer Optimierung der Leistung der Absorbermaske in der Strahlentherapie beitragen, insbesondere durch die Fokussierung der Absorbermaske .

Eine Ausführungsform sieht vor, dass die Anlage eine Kippvorrichtung aufweist und dass die Auftragungseinheit sowie die Kippvorrichtung derart angeordnet sind, dass mittels der Kippvorrichtung ein Kippwinkel der Auftragungseinheit relativ zu einer bereits aufgetragenen Lage veränderbar ist. Die Kippvorrichtung kann ein mechanisches System sein, das es ermöglicht, die Auftragungseinheit um eine oder mehrere Achsen zu neigen. Diese Kippvorrichtung kann beispielsweise aus Gelenken, Scharnieren oder Schwenkvorrichtungen bestehen, die eine präzise Kontrolle über den Winkel der Auftragungseinheit erlauben. Die Anordnung der Auftragungseinheit und der Kippvorrichtung kann so gestaltet sein, dass eine Veränderung des Kippwinkels der Auftragungseinheit relativ zu einer bereits aufgetragenen Lage möglich ist. Dies bedeutet, dass die Auftragungseinheit in verschiedenen Winkeln zur Oberfläche der zuletzt aufgetragenen Materialschicht positioniert werden kann. Durch die Kippachse der Kippvorrichtung kann die beispielsweise auf einer vertikal beweglichen Platte entstehende Absorbermaske mit unterschiedlichen Kippwinkeln relativ zu der Fläche, in der sich die Auftragungseinheit bewegt, mittels der Kippvorrichtung verkippbar sein. Alternativ oder zusätzlich kann die Auftragungseinheit gegen die Horizontale mittels der Kippvorrichtung verkippbar sein. Die Veränderung des Kippwinkels kann durch eine manuelle Einstellung oder durch eine automatisierte Steuerung erfolgen. Eine automatisierte Steuerung kann beispielsweise durch Servomotoren oder Stellantriebe realisiert werden, die mit einer Steuerungseinheit verbunden sind. Diese Steuerungseinheit kann den Kippwinkel basierend auf vorprogrammierten Parametern oder in Echtzeit während des Fertigungsprozesses anpassen. Der veränderbare Kippwinkel der Auftragungseinheit kann es ermöglichen, fokussierte Absorbermasken herzustellen. Bei einer fokussierten Absorbermaske sind die absorbierenden Strukturen so ausgerichtet, dass sie auf einen bestimmten Punkt, typischerweise die Röntgenquelle, ausgerichtet sind. Dies kann durch eine graduelle Änderung des Winkels der aufgetragenen Lagen erreicht werden.

Durch die Verwendung der Kippvorrichtung kann der Winkel jeder aufgetragenen Lage präzise gesteuert werden. Beispielsweise kann der Kippwinkel so eingestellt werden, dass die Lagen im Zentrum der Maske parallel zur Oberfläche aufgetragen werden, während die Lagen zum Rand hin zunehmend geneigt sind. Dies kann zu einer verbesserten Effizienz der Absorbermaske führen, da die absorbierenden Strukturen optimal zur Röntgenquelle ausgerichtet sind. Zudem kann die Kippvorrichtung dazu beitragen, die Oberflächenqualität der hergestellten Absorbermaske zu verbessern. Durch Anpassung des Kippwinkels kann der Materialauftrag so gesteuert werden, dass Überhänge oder Unterschneidungen vermieden werden, was zu einer glatteren Oberfläche und einer verbesserten strukturellen Integrität der Maske führen kann. Die Verwendung einer Kippvorrichtung in der Anlage zur additiven Herstellung einer Absorbermaske kann eine erhöhte Flexibilität und Präzision im Herstellungsprozess ermöglichen. Dies kann zu einer verbesserten Leistung der Absorbermaske in der Strahlentherapie beitragen, indem die Geometrie und Ausrichtung der absorbierenden Strukturen optimal an die spezifischen Anforderungen der jeweiligen Anwendung angepasst werden können.

Eine erfindungsgemäße Verwendung einer Anlage mit einer Auftragungseinheit zur additiven Herstellung einer Absorbermaske ist ebenfalls Teil der Erfindung. Diese Verwendung bezieht sich auf den Einsatz der zuvor beschriebenen Anlage oder anderer Anlagen mit einer Auftragungseinheit zur additiven Herstellung für das Verfahren zur Herstellung der erfindungsgemäßen Absorbermaske. Jede Anlage mit einer solchen Auftragungseinheit kann typischerweise dazu verwendet werden, die verschiedenen Schichten der Absorbermaske präzise und effizient aufzubauen.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Ausführungsbeispiele der Erfindung sind in den Zeichnungen dargestellt und werden im Folgenden näher beschrieben. In unterschiedlichen Figuren werden für gleiche Merkmale die gleichen Bezugszeichen verwendet.

Es zeigen:
- Fig. 1: eine schematische Darstellung einer ersten Variante einer erfindungsgemäßen Absorbermaske;
- Fig. 2: eine perspektivische Ansicht einer zweiten Variante einer erfindungsgemäßen Absorbermaske;
- Fig. 3: eine perspektivische Ansicht einer dritten Variante einer erfindungsgemäßen Absorbermaske;
- Fig. 4: eine schematische Darstellung eines ersten Ausführungsbeispiels der ersten Variante der erfindungsgemäßen Absorbermaske;
- Fig. 5: eine schematische Darstellung eines zweiten Ausführungsbeispiels der ersten Variante der erfindungsgemäßen Absorbermaske;
- Fig. 6: eine schematische Darstellung einer erfindungsgemäßen Anordnung für eine Strahlentherapie;
- Fig. 7: eine schematische Darstellung eines ersten Ausführungsbeispiels der Anordnung für eine Strahlentherapie;
- Fig. 8: ein Flussdiagramm eines erfindungsgemäßen Verfahrens;
- Fig. 9: eine perspektivische Ansicht einer erfindungsgemäßen Anlage zur additiven Herstellung einer Absorbermaske;
- Fig. 10: eine schematische Seitenansicht eines ersten Ausführungsbeispiels der Anlage und
- Fig. 11: eine schematische Seitenansicht eines zweiten Ausführungsbeispiels der Anlage.

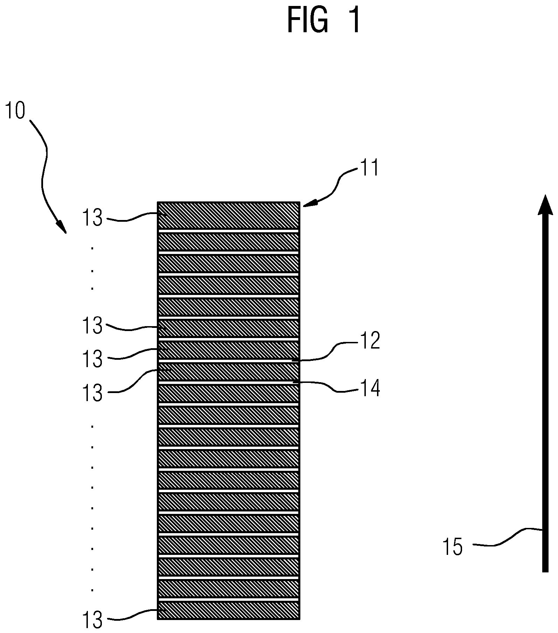

Fig. 1 zeigt eine schematische Darstellung einer ersten Variante einer erfindungsgemäßen Absorbermaske 10 als Querschnitt in Breitenrichtung. Die Darstellung der Absorbermaske 10 ist nicht maßstabsgetreu.

Die erfindungsgemäße Absorbermaske 10 zum Formen einer Vielzahl von therapeutischen Röntgenstrahlen, insbesondere für eine Strahlentherapie eines Patienten P, weist einen Schichtstapel 11 auf. Der Schichtstapel 11 weist mehrere Röntgenstrahlen-absorbierende Schichten 13 auf. Die mehreren Röntgenstrahlen-absorbierenden Schichten 13 weisen jeweils mindestens eine flächige Lage auf. Benachbarte Lagen des Schichtstapels 11 sind mittels eines additiven Herstellungsverfahrens in Stapelrichtung flächig aufeinander aufgetragen. Der Pfeil kennzeichnet die Stapelrichtung 15.

Fig. 1 zeigt die erste Variante der erfindungsgemäßen Absorbermaske 10, wobei zwischen den mehreren Röntgenstrahlen-absorbierenden Schichten 13 mindestens eine Röntgenstrahlen-transparente Schicht 12, 14, aber kein sich in Breitenrichtung erstreckender Röntgenstrahlen-absorbierender Streifen angeordnet ist. Vorzugsweise sind zwischen allen mehreren Röntgenstrahlen-absorbierenden Schichten 13 Röntgenstrahlen-transparente Schichten 12, 14 angeordnet.

Die mindestens eine Röntgenstrahlen-transparente Schicht 12, 14 kann aus einer ersten Materialzusammensetzung ausgebildet sein, wobei die erste Materialzusammensetzung Glashohlkugeln und/oder ein Aerogel aufweist. Die mehreren Röntgenstrahlen-absorbierenden Schichten 13 können aus einer zweiten Materialzusammensetzung ausgebildet sein, wobei die zweite Materialzusammensetzung Blei, Tantal, Wolfram, Rhenium, Osmium, Iridium, Bismut, Platin, Thallium, Quecksilber oder Gold aufweist. Die erste Materialzusammensetzung kann eine Kunststoff-Matrix aufweisen und die zweite Materialzusammensetzung kann die gleiche Kunststoff-Matrix aufweisen. Die erste Materialzusammensetzung und die zweite Materialzusammensetzung können alternative unterschiedliche Kunststoff-Matrizen aufweisen.

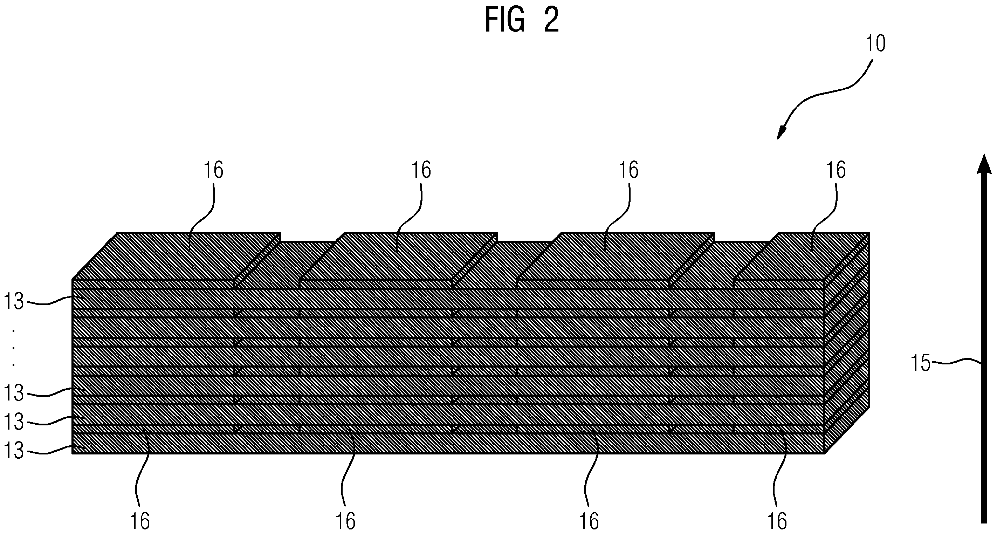

Fig. 2 zeigt eine perspektivische Ansicht einer zweiten Variante einer erfindungsgemäßen Absorbermaske 10. Im Vergleich zur Fig. 1 ist die Darstellung im Wesentlichen um 90° gedreht, so dass die Breitenrichtung in die Bildebene hineingeht. Die Darstellung der Absorbermaske 10 ist nicht maßstabsgetreu und insbesondere nicht repräsentativ in Hinblick auf die Anzahl der Streifen auf die Länge der Absorbermaske.

Fig. 2 zeigt die zweite Variante der erfindungsgemäßen Absorbermaske 10, wobei zwischen den mehreren Röntgenstrahlen-absorbierenden Schichten 13 mindestens ein sich in Breitenrichtung erstreckender Röntgenstrahlen-absorbierender Streifen 16, aber keine Röntgenstrahlen-transparente Schicht 12, 14 angeordnet ist. Vorzugsweise sind zwischen allen mehreren Röntgenstrahlen-absorbierenden Schichten 13 mehrere Röntgenstrahlen-absorbierende Streifen 16, auch je Schicht, angeordnet.

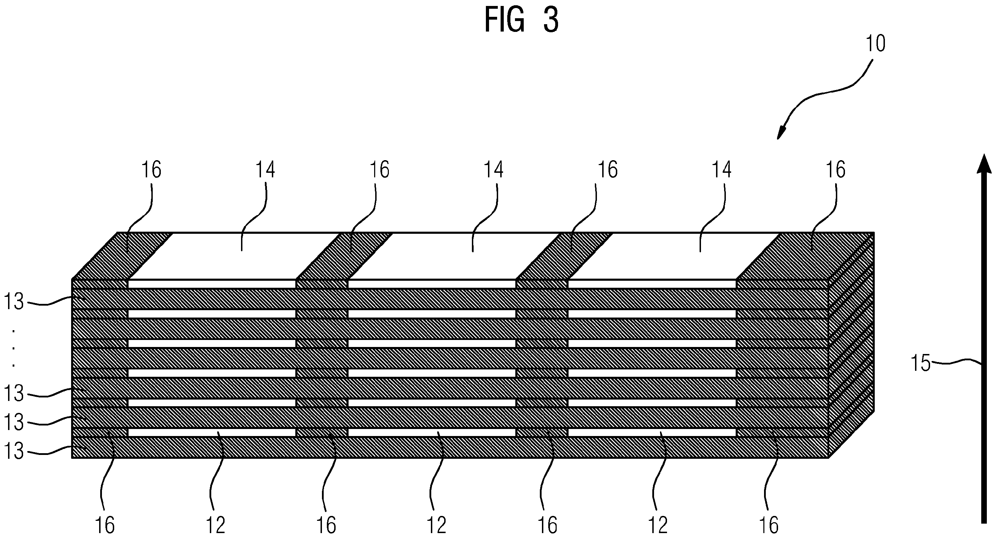

Fig. 3 zeigt eine perspektivische Ansicht einer dritten Variante einer erfindungsgemäßen Absorbermaske 10. Im Vergleich zur Fig. 1 ist die Darstellung im Wesentlichen um 90° gedreht, so dass die Breitenrichtung in die Bildebene hineingeht. Die Darstellung der Absorbermaske 10 ist nicht maßstabsgetreu und insbesondere nicht repräsentativ in Hinblick auf die Anzahl der Streifen auf die Länge der Absorbermaske.

Fig. 3 zeigt die dritte Variante der erfindungsgemäßen Absorbermaske 10, wobei zwischen den mehreren Röntgenstrahlen-absorbierenden Schichten 13 mindestens ein sich in Breitenrichtung erstreckender Röntgenstrahlen-absorbierender Streifen 16 und mindestens eine Röntgenstrahlen-transparente Schicht 12, 14 angeordnet ist. Vorzugsweise sind zwischen allen mehreren Röntgenstrahlen-absorbierenden Schichten 13 mehrere Röntgenstrahlen-absorbierende Streifen 16, auch je Schicht, angeordnet. Vorzugsweise sind zwischen allen mehreren Röntgenstrahlen-absorbierenden Schichten 13 Röntgenstrahlen-transparente Schichten 12, 14 angeordnet.

Fig. 2 und Fig. 3 zeigen im Vergleich also insbesondere, dass die Ausdehnung der Streifen 16 in Längenausdehnung der Absorbermaske 10 variieren kann und dass in die Abstände zwischen den Streifen Röntgenstrahlen-transparentes Material eingefüllt sein kann oder diese materialfrei sind. Diese Abstände, auch Öffnungen oder Hohlräume, dienen der Formung der Vielzahl an therapeutischen Röntgenstrahlen. Im Fall der Fig. 2 und 3 mit den Streifen, sind die therapeutischen Röntgenstrahlen eher nadelförmig und im Fall der Fig. 1 eher streifenförmig.

Fig. 3 zeigt ferner, dass eine Dicke einer der mehreren Röntgenstrahlen-absorbierenden Schichten 13 größer ist als eine Dicke der Röntgenstrahlen-transparenten Schicht 12, 14 bzw. des mindestens einen Röntgenstrahlen-absorbierenden Streifens 16. Die Röntgenstrahlen-absorbierenden Streifen 16 von benachbarten Lagen setzen sich zu einer regelmäßigen Struktur zusammen.

Eine Ausführungsform sieht vor, dass ein Abstand innerhalb einer Lage zwischen Röntgenstrahlen-absorbierenden Streifen 16 kleiner gleich 10 mm, vorzugsweise kleiner gleich 1 mm, beträgt. Alternativ oder zusätzlich kann ein Abstand innerhalb einer Lage zwischen Röntgenstrahlen-absorbierenden Streifen größer gleich 0,01 mm, vorzugsweise größer gleich 0,1 mm, betragen. Insbesondere kann ein Verhältnis innerhalb einer Lage zwischen einer Länge eines Röntgenstrahlen-absorbierende Streifen und einem benachbarten Abstand größer gleich 1, vorzugsweise größer gleich 2, betragen. Der Abstand zwischen zwei Röntgenstrahlen-absorbierenden Streifen kann insbesondere dann größer sein, wenn zwischen diesen Röntgenstrahlen-absorbierenden Streifen Röntgenstrahlen-transparentes Material angeordnet ist.

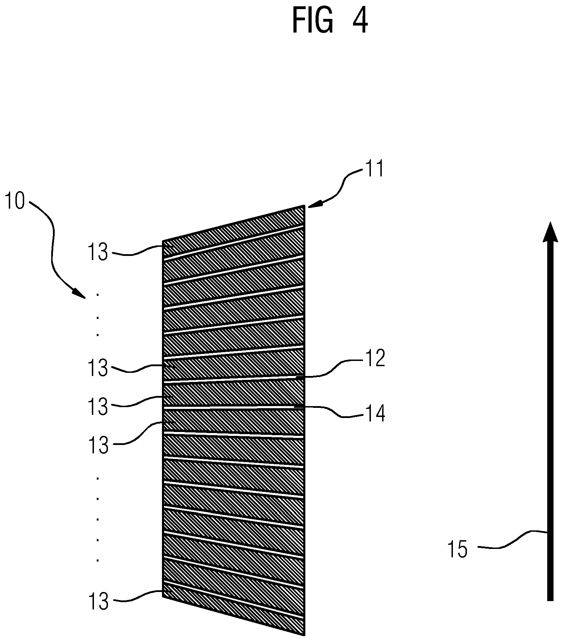

Fig. 4 zeigt eine schematische Darstellung eines ersten Ausführungsbeispiels der ersten Variante der erfindungsgemäßen Absorbermaske 10 als Schnitt in Breitenrichtung. Die Darstellung der Absorbermaske 10 ist nicht maßstabsgetreu.

Der Querschnitt mindestens einer Lage in Breitenrichtung ist trapezförmig ausgebildet. Dadurch ist die Absorbermaske 10 eine fokussierte Absorbermaske. Die Absorbermaske 10 kann insbesondere auf den Zielbereich im Gewebe des Patienten P fokussiert sein. Die Trapezform der mehreren Röntgenstrahlen-absorbierenden Schichten 13 definiert insbesondere einen Fokuspunkt.

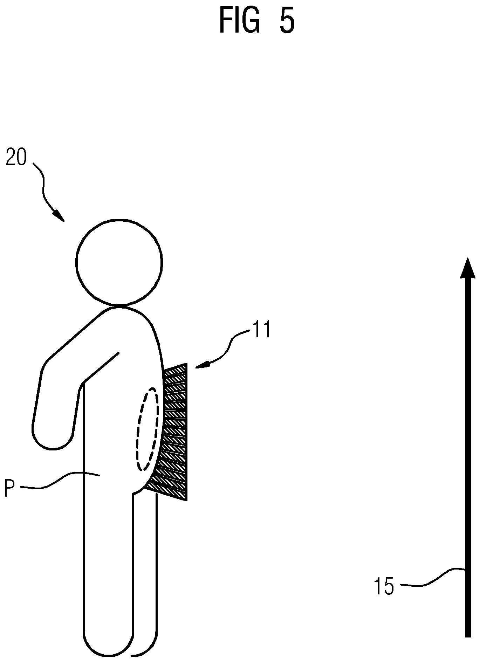

Fig. 5 zeigt eine schematische Darstellung eines zweiten Ausführungsbeispiels der ersten Variante der erfindungsgemäßen Absorbermaske 10 als Schnitt in Breitenrichtung. Die Darstellung der Absorbermaske 10 ist nicht maßstabsgetreu.

Der Schichtstapel 11 ist in Abhängigkeit von einer Kontur des Patienten P derart gefertigt, dass eine Seite der Absorbermaske 11 im Wesentlichen das Negativ der Kontur des Patienten P aufweist. Im Vergleich zur Fig. 4 ist die Oberseite der Absorbermaske derart geformt, dass sie passgenau auf den Patienten P passt. Innerhalb des Patienten P ist der Zielbereich mit einem gestrichelten Kreis gekennzeichnet. Vorzugsweise liegt der Fokuspunkt der mehreren Röntgenstrahlen-absorbierenden Schichten 13 im Zielbereich des Gewebes des Patienten P.

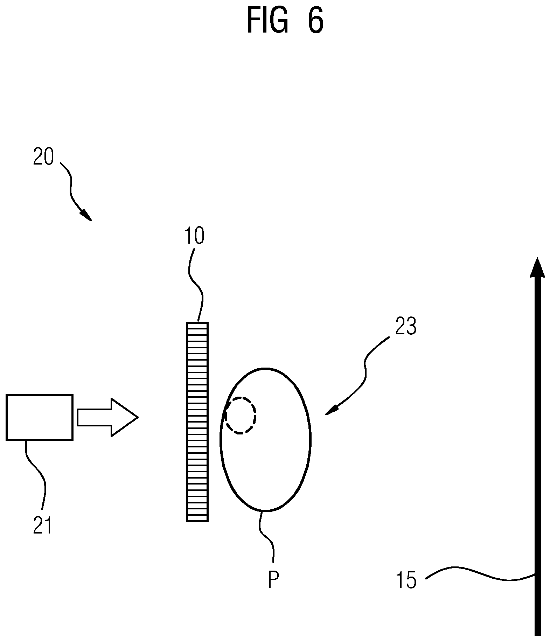

Fig. 6 zeigt eine schematische Darstellung einer erfindungsgemäßen Anordnung 20. Die Darstellung ist nicht maßstabsgetreu.

Die Anordnung 20 für eine Strahlentherapie eines Patienten weist eine Absorbermaske 10, eine therapeutische Röntgenstrahlenquelle 21 und einen Therapiebereich 23 auf. Die Absorbermaske 10 ist zwischen dem Therapiebereich 23 und der therapeutischen Röntgenstrahlenquelle 21 angeordnet und derart ausgerichtet, dass die Stapelrichtung 15 des Schichtstapels 11 im Wesentlichen senkrecht zur Röntgenstrahlung der therapeutischen Röntgenstrahlenquelle 21 ist. Innerhalb des Patienten P ist der Zielbereich mit einem gestrichelten Kreis gekennzeichnet.

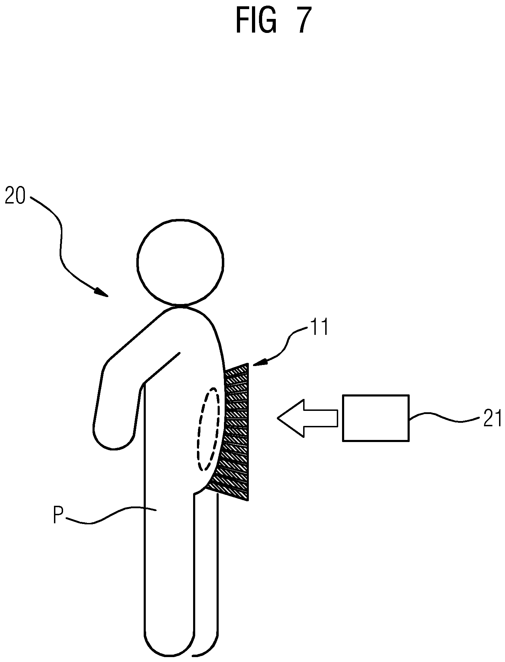

Fig. 7 zeigt eine schematische Darstellung eines ersten Ausführungsbeispiels der erfindungsgemäßen Anordnung 20. Die Darstellung ist nicht maßstabsgetreu.

Die Absorbermaske 10 ist auf der Kontur des Patienten angeordnet. Eine Position des Fokuspunkts der mehreren Röntgenstrahlen-absorbierenden Schichten 13 liegt nahe an der Kontur des Patienten P.

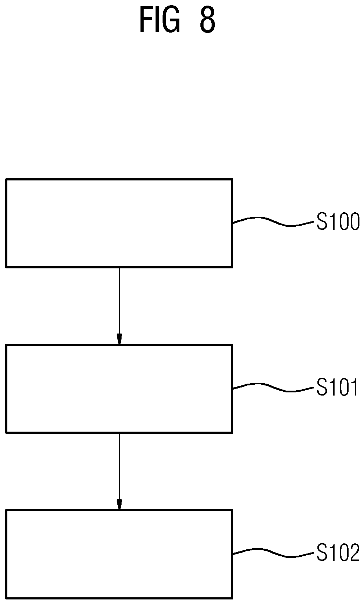

Fig. 8 zeigt ein Flussdiagramm eines erfindungsgemäßen Verfahrens mit den Schritten S100 bis S102. Das in Fig. 8 dargestellte Verfahren bzw. einzelne oder alle Verfahrensschritte S100 bis S102 können wiederholt durchgeführt werden, um einen Schichtstapel 11 mit mehreren Röntgenstrahlen-transparenten Schichten 13 zu erzeugen, wobei zwischen den mehreren Röntgenstrahlen-absorbierenden Schichten 13 mindestens eine Röntgenstrahlen-transparente Schicht 12, 14 und/oder mindestens ein sich in Breitenrichtung erstreckender Röntgenstrahlen-absorbierender Streifen 16 angeordnet ist.

Verfahrensschritte S100 bis S102 kennzeichnen ein Auftragen mittels eines additiven Herstellungsverfahrens von Lagen flächig aufeinander in Stapelrichtung zu einem Schichtstapel derart,
- dass der Schichtstapel 11 mehrere Röntgenstrahlen-absorbierende Schichten 13 aufweist und
- dass der Schichtstapel 11 zwischen den mehreren Röntgenstrahlen-absorbierenden Schichten 13 mindestens eine Röntgenstrahlen-transparente Schicht 12, 14 und/oder mindestens einen sich in Breitenrichtung erstreckenden Röntgenstrahlen-absorbierenden Streifen 16 aufweist. Bei einem oder jedem der Verfahrensschritte S100 bis S102 kann grundsätzlich eine Dicke einer Lage des Schichtstapels 11 mittels des additiven Herstellungsverfahrens in Breitenrichtung variiert werden, insbesondere um einen Querschnitt dieser Lage in Breitenrichtung trapezförmig auszubilden.

Die Röntgenstrahlen-transparenten Schichten 12, 14 können oder werden aus einer ersten Materialzusammensetzung und die mehreren Röntgenstrahlen-absorbierenden Schichten 13 sind oder werden aus einer zweiten Materialzusammensetzung ausgebildet, wobei die erste Materialzusammensetzung und die zweite Materialzusammensetzung bezüglich eines Anteils eines Materials voneinander abweichend ausgebildet sind. Die erste Materialzusammensetzung kann Glashohlkugeln und/oder ein Aerogel aufweisen. Alternativ oder zusätzlich kann die zweite Materialzusammensetzung Blei, Tantal, bevorzugt Wolfram, Rhenium, Osmium, Iridium, Bismut, Platin, Thallium, Quecksilber oder Gold aufweisen. Es ist denkbar, dass die erste Materialzusammensetzung eine Kunststoff-Matrix aufweist und die zweite Materialzusammensetzung die gleiche Kunststoff-Matrix aufweist.

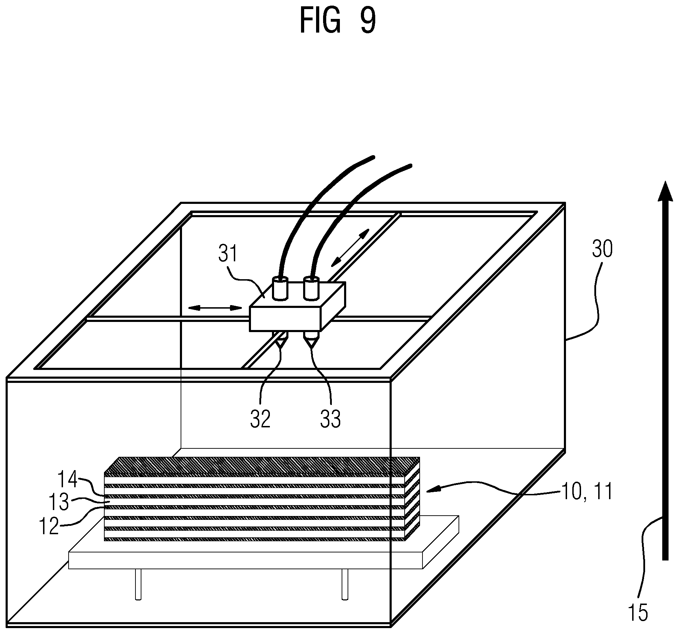

Fig. 9 zeigt eine perspektivische Ansicht einer erfindungsgemäßen Anlage 30 zur additiven Herstellung einer Absorbermaske 10.

Die Anlage 30 weist eine Auftragungseinheit 31 auf, welche dazu ausgebildet ist, ein erfindungsgemäßes Herstellungsverfahren zur Herstellung der Absorbermaske 10 durchzuführen. Die Anlage 30 kann mehrere Komponenten aufweisen, die in einer vertikalen Konfiguration angeordnet sein können. Am unteren Teil der Anlage 30 kann eine Platte angeordnet sein wie in Fig. 9 dargestellt, die den Schichtstapel 11 tragen und insbesondere vertikal bewegen kann. Dies kann eine Anpassung des Abstands zwischen der Auftragungseinheit 31 und dem Schichtstapel 11 ermöglichen, wenn Lagen aufgetragen werden. Oberhalb des Schichtstapels 11 kann wie in Fig. 9 dargestellt eine Rahmenstruktur angeordnet sein. Diese Rahmenstruktur kann parallel zur Platte positioniert sein und in einer horizontalen Ebene beweglich sein, was durch einen Doppelpfeil angedeutet ist. An der Rahmenstruktur kann die Auftragungseinheit 31 montiert sein. Die Anlage 30 der Fig. 8 ist insbesondere als "core XY"-Anordnung ausgeführt, was eine effiziente additive Fertigung ermöglichen kann.

Die Auftragungseinheit 31 der Fig. 9 weist zwei Auftragungsdüsen 32, 33 auf, die zum Auftragen von Röntgenstrahlen-transparenten Material und Röntgenstrahlen-absorbierenden Material zur Bildung der Schichten 12, 13, 14 bzw. der Streifen 16 der Absorbermaske 10 verwendet werden können. Die Auftragungseinheit 31 kann entlang der Rahmenstruktur beweglich sein, was das Auftragen von Material über die gesamte Oberfläche des darunter liegenden Schichtstapels 11 ermöglichen kann. Die Auftragungseinheit 31 kann über mehrere Kabel oder Rohre verbunden sein. Diese können zur Versorgung der Auftragungseinheit 31 mit Materialien, Energie oder Steuersignalen dienen. Die Auftragungsdüsen 32, 33 können jeweils einen ovalen oder rechteckigen Querschnitt aufweisen.

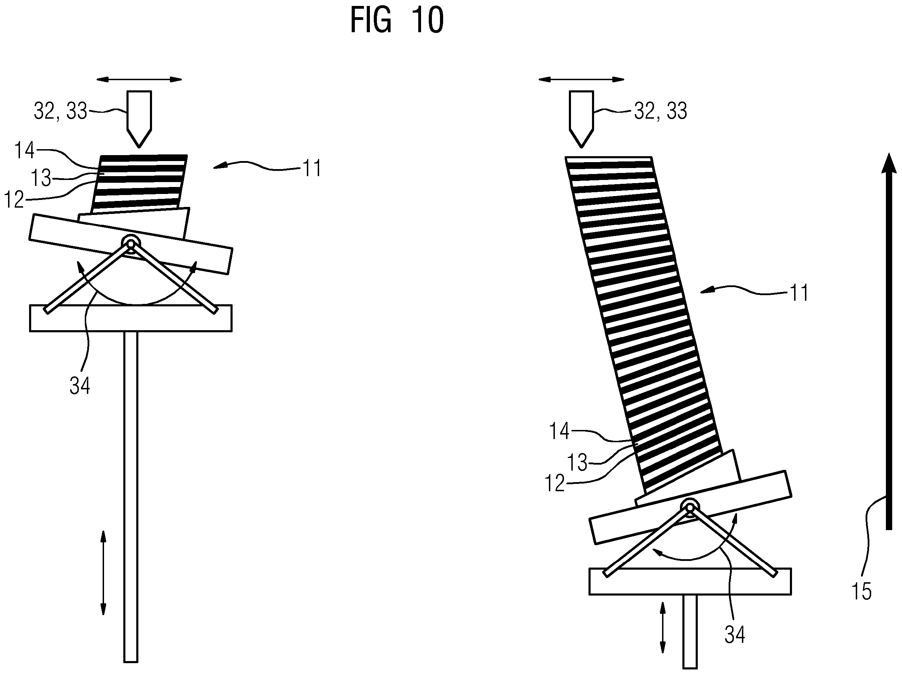

Fig. 10 zeigt eine schematische Seitenansicht eines ersten Ausführungsbeispiels der Anlage. In der Fig. 10 sind zwei Konfigurationen der Anlage 30 zu zwei unterschiedlichen Zeitpunkten der Herstellung des Schichtstapels 11 enthalten.

Die Anlage 30 der Fig. 10 weist eine Kippvorrichtung 34 unterhalb des Schichtstapels 11 auf. Die Kippvorrichtung 34 ist dazu ausgebildet, den Kippwinkel des Schichtstapels 11 relativ zu den Auftragungsdüsen 32, 33 einzustellen. Die Kippvorrichtung 34 ist insbesondere dazu ausgebildet, den Kippwinkel der Auftragungseinheit 31 relativ zu einer bereits aufgetragenen Lage zu verändern.

In der linken Konfiguration ist die Platte, auf welcher der Schichtstapel 11 Lage für Lage aufgetragen wird, nach rechts geneigt. Die Auftragungsdüsen 32, 33 sind dazu ausgebildet, eine Lage auf eine bereits gefertigte Lage flächig aufzutragen. In der rechten Konfiguration hat die Kippvorrichtung 34 den Winkel des Schichtstapels 11 derart angepasst, so dass die Platte nach links geneigt ist. Die Kippvorrichtung 34 kann insbesondere für jede Lage einen unterschiedlichen Kippwinkel einstellen. Der Kippwinkel variiert typischerweise graduell von Lage zu Lage entlang der Stapelrichtung 15.

Die Auftragungsdüsen 32, 33 sind mit horizontalen Pfeilen dargestellt, was ihre seitliche Bewegung während des Auftragungsprozesses andeutet. Der Schichtstapel 11 zeigt in beiden Konfigurationen abwechselnd helle und dunkle Schichten, die die Röntgenstrahlen-transparenten Schichten 12, 14 und Röntgenstrahlen-absorbierenden Schichten 13 darstellen können.

Die Konstruktion der Anlage 30 kann die Herstellung von fokussierten Absorbermasken 10 ermöglichen, indem der Auftragungswinkel durch die Kippvorrichtung 34 eingestellt werden kann. Dies kann die Produktion von Masken mit variierenden Geometrien ermöglichen, um verschiedene Anforderungen der Strahlentherapie zu erfüllen. Diese Ausführungsform ist insbesondere vorteilhaft, wenn eine Dicke einer Lage oder jeder Lage des Schichtstapels 11 mittels des additiven Herstellungsverfahrens in Breitenrichtung variiert wird. Dadurch kann insbesondere eine fokussierte Absorbermaske gefertigt werden, bei der ein Querschnitt einer Lage oder jeder Lage in Breitenrichtung trapezförmig ausgebildet ist.

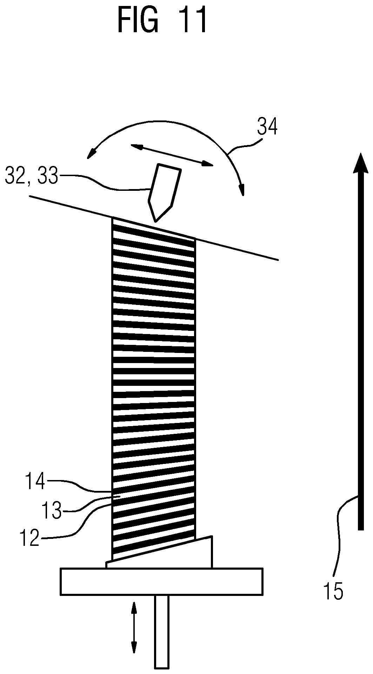

Fig. 11 zeigt eine schematische Seitenansicht eines zweiten Ausführungsbeispiels der Anlage 30. Das Ausführungsbeispiel der Fig. 11 kann alternativ oder zusätzlich zum Ausführungsbeispiel der Fig. 10 sein.

Die Anlage 30 der Fig. 11 weist eine Kippvorrichtung 34 auf, welche dazu ausgebildet ist, mittels der Kippvorrichtung 34 einen Kippwinkel der Auftragungseinheit 31 relativ zu einer bereits aufgetragenen Lage zu verändern. In diesem Ausführungsbeispiel ist die Kippvorrichtung 34 beispielsweise zwischen der Rahmenstruktur und der Auftragungseinheit 31 angeordnet, so dass die Auftragungseinheit 31, insbesondere die beiden Auftragungsdüsen 32, 33 in Bezug auf die Stapelrichtung 15 verkippbar sind. Im Vergleich dazu sind die Auftragungsdüsen 32, 33 der Fig. 10 in Bezug auf die Stapelrichtung 15 nicht verkippbar, sondern die Platte mit dem Schichtstapel 11 sind in Bezug auf die Stapelrichtung 15 verkippbar.

Unabhängig vom grammatikalischen Geschlecht eines bestimmten Begriffes sind Personen mit männlicher, weiblicher oder anderer Geschlechteridentität mit umfasst.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Absorbermaske (10) zum Formen einer Vielzahl von therapeutischen Röntgenstrahlen, insbesondere für eine Strahlentherapie eines Patienten (P), aufweisend
- einen Schichtstapel (11),
- wobei der Schichtstapel (11) mehrere Röntgenstrahlen-absorbierende Schichten (13) aufweist,
- wobei die mehreren Röntgenstrahlen-absorbierenden Schichten (13) jeweils mindestens eine flächige Lage aufweisen,
- wobei benachbarte Lagen des Schichtstapels (11) mittels eines additiven Herstellungsverfahrens in Stapelrichtung (15) flächig aufeinander aufgetragen sind,
- wobei zwischen den mehreren Röntgenstrahlen-absorbierenden Schichten (13) mindestens eine Röntgenstrahlen-transparente Schicht (12, 14) und/oder mindestens ein sich in Breitenrichtung erstreckender Röntgenstrahlen-absorbierender Streifen (16) angeordnet ist.

2. Absorbermaske (10) nach Anspruch 1,
wobei eine Dicke einer der mehreren Röntgenstrahlen-absorbierenden Schichten (13) größer ist als eine Dicke der Röntgenstrahlen-transparenten Schicht (12, 14) bzw. des mindestens einen Röntgenstrahlen-absorbierenden Streifens (16).

3. Absorbermaske (10) nach einem der vorhergehenden Ansprüche,
wobei der Schichtstapel (11) in Abhängigkeit von einer Kontur eines Patienten (P) derart gefertigt ist, dass eine Seite der Absorbermaske (11) im Wesentlichen das Negativ der Kontur des Patienten (P) aufweist.

4. Absorbermaske (10) nach einem der vorhergehenden Ansprüche,
wobei die mindestens eine Röntgenstrahlen-transparente Schicht (12, 14) aus einer ersten Materialzusammensetzung ausgebildet ist, wobei die erste Materialzusammensetzung Glashohlkugeln und/oder ein Aerogel aufweist.

5. Absorbermaske (10) nach einem der vorhergehenden Ansprüche,
wobei die mehreren Röntgenstrahlen-absorbierenden Schichten (13) aus einer zweiten Materialzusammensetzung ausgebildet sind, wobei die zweite Materialzusammensetzung Blei, Tantal, Wolfram, Rhenium, Osmium, Iridium, Bismut, Platin, Thallium, Quecksilber oder Gold aufweist.

6. Absorbermaske (10) nach einem der Ansprüche 4 bis 5,
wobei die erste Materialzusammensetzung eine Kunststoff-Matrix aufweist und die zweite Materialzusammensetzung die gleiche Kunststoff-Matrix aufweist.

7. Absorbermaske (10) nach einem der vorhergehenden Ansprüche,
wobei ein Abstand innerhalb einer Lage zwischen Röntgenstrahlen-absorbierenden Streifen (16) kleiner gleich 10 mm, vorzugsweise kleiner gleich 1 mm, beträgt.

8. Absorbermaske (10) nach einem der vorhergehenden Ansprüche,
wobei ein Abstand innerhalb einer Lage zwischen Röntgenstrahlen-absorbierenden Streifen (16) größer gleich 0,01 mm, vorzugsweise größer gleich 0,1 mm, beträgt.

9. Absorbermaske (10) nach einem der vorhergehenden Ansprüche,
wobei sich Röntgenstrahlen-absorbierende Streifen (16) von benachbarten Lagen zu einer regelmäßigen Struktur zusammensetzen.

10. Absorbermaske (10) nach einem der vorhergehenden Ansprüche,
wobei ein Verhältnis innerhalb einer Lage zwischen einer Länge eines Röntgenstrahlen-absorbierende Streifen (16) und einem benachbarten Abstand größer gleich 1, vorzugsweise größer gleich 2, beträgt.

11. Absorbermaske (10) nach einem der vorhergehenden Ansprüche,
wobei ein Querschnitt einer Lage in Breitenrichtung trapezförmig ausgebildet ist.

12. Anordnung (20) für eine Strahlentherapie eines Patienten (P), aufweisend
- eine Absorbermaske (10) nach einem der vorhergehenden Ansprüche,
- eine therapeutische Röntgenstrahlenquelle (21)
und
- einen Therapiebereich (23),
- wobei die Absorbermaske (10) zwischen dem Therapiebereich (23) und der therapeutischen Röntgenstrahlenquelle (21) angeordnet und derart ausgerichtet ist, dass die Stapelrichtung (15) des Schichtstapels (11) im Wesentlichen senkrecht zur Röntgenstrahlung der therapeutischen Röntgenstrahlenquelle (21) ist.

13. Anordnung (20) nach Anspruch 12,
wobei die Absorbermaske (10) nach den Ansprüchen 3 und 11 ausgebildet ist, wobei die Absorbermaske (10) auf der Kontur des Patienten angeordnet ist und wobei eine Position des Fokuspunkts der mehreren Röntgenstrahlen-absorbierenden Schichten (13) nahe an der Kontur des Patienten liegt.

14. Verfahren zur Herstellung einer Absorbermaske (10) zum Formen einer Vielzahl von therapeutischen Röntgenstrahlen, insbesondere für eine Strahlentherapie eines Patienten nach einem der Ansprüche 1 bis 11, umfassend die Schritte:
- Auftragen (S100, S101, S102) mittels eines additiven Herstellungsverfahrens von Lagen flächig aufeinander in Stapelrichtung zu einem Schichtstapel derart,
- dass der Schichtstapel (11) mehrere Röntgenstrahlen-absorbierende Schichten (13) aufweist und
- dass der Schichtstapel (11) zwischen den mehreren Röntgenstrahlen-absorbierenden Schichten (13) mindestens eine Röntgenstrahlen-transparente Schicht (12, 14) und/oder mindestens einen sich in Breitenrichtung erstreckenden Röntgenstrahlen-absorbierenden Streifen (16) aufweist.

15. Anlage (30) zur additiven Herstellung einer Absorbermaske (10) zum Formen einer Vielzahl von therapeutischen Röntgenstrahlen, insbesondere für eine Strahlentherapie eines Patienten nach einem der Ansprüche 1 bis 11, aufweisend eine Auftragungseinheit (31), welche dazu ausgebildet ist, ein Herstellungsverfahren nach Anspruch 14 durchzuführen.
